# FASCICULE DE BREVET EUROPEEN

(11) **EP 4 278 324 B1**
(45) Date de publication et mention de la délivrance du brevet: **02.12.2026**
(21) Numéro de dépôt: 22702765.3
(22) Date de dépôt: 13.01.2022
(51) Int. Cl.: G06T 7/246

(54) **PROCÉDÉ DE DÉTECTION ET DE SUIVI DANS UN FLUX VIDÉO D'UN VISAGE D'UN INDIVIDU PORTANT UNE PAIRE DE LUNETTES**
VERFAHREN ZUR ERKENNUNG UND ÜBERWACHUNG DES GESICHTS EINER PERSON MIT BRILLEN IN EINEM VIDEOSTROM
METHOD FOR DETECTING AND MONITORING THE FACE OF A PERSON WEARING GLASSES IN A VIDEO STREAM

(30) Priorité: 13.01.2021 FR 2100297
(43) Date de publication de la demande: 22.11.2023
(73) Titulaire: Fittingbox, 31670 Labege (FR)
(72) Inventeur: CHOUKROUN, Ariel, 31650 SAINT ORENS DE GAMEVILLE (FR); GUENARD, Jérome, 31500 TOULOUSE (FR)
(74) Mandataire: Santarelli
(86) Numéro de dépôt international: PCT/FR2022/050067
(87) Numéro de publication internationale: WO 2022/153009

(56) Documents cités:
- US-A1- 2017 068 121
- US-A1- 2019 108 687
- MANINCHEDDA FABIO ET AL: "Fast 3D Reconstruction of Faces with Glasses", 2017 IEEE CONFERENCE ON COMPUTER VISION AND PATTERN RECOGNITION (CVPR), IEEE COMPUTER SOCIETY, US, 21 July 2017 (2017-07-21), pages 4608 - 4617, XP033249816, ISSN: 1063-6919, [retrieved on 20171106], DOI: 10.1109/CVPR.2017.490

## Description

### DOMAINE TECHNIQUE DE L'INVENTION

Le domaine de l'invention est celui de l'analyse d'images.

Plus précisément, l'invention concerne un procédé de détection et de suivi dans un flux vidéo d'un visage d'un individu portant une paire de lunettes.

L'invention trouve notamment des applications pour l'essayage virtuel d'une paire de lunettes. L'invention trouve également des applications en réalité augmentée ou diminuée sur un visage porteur de lunettes, avec notamment l'occultation à l'image de la paire de lunettes portée par l'individu, combiné ou non avec l'ajout de lentilles, de bijoux et/ou de maquillage. L'invention trouve également des applications pour la prise de mesure ophtalmique (PD, monoPD, hauteurs, etc.) sur une paire de lunettes portée réellement ou virtuellement par un individu.

La demande US2017/068121 est un exemple d'une telle méthode pour l'essayage virtuel de lunettes.

### ÉTAT DE LA TECHNIQUE

Il est connu de l'art antérieur des techniques qui permettent de détecter et de suivre un visage d'un individu dans un flux vidéo.

Ces techniques sont généralement basées sur la détection et le suivi de points caractéristiques du visage, tels qu'un coin des yeux, un nez ou un coin d'une bouche. La qualité de la détection du visage est généralement fonction du nombre et de la position des points caractéristiques utilisés.

Ces techniques sont généralement fiables pour détecter et suivre un visage d'un individu sans accessoire dans un flux vidéo.

De telles techniques sont notamment décrites dans le brevet français publié sous le numéro FR 2955409 et dans la demande internationale de brevet publiée sous le numéro WO 2016/135078, de la société déposant la présente demande de brevet.

Toutefois, lorsque l'individu porte une paire de lunettes comprenant des verres correcteurs, la qualité de la détection du visage a tendance à se dégrader car une partie des points caractéristiques utilisés au cours de la détection, généralement les coins des yeux, sont généralement déformés par les verres assemblés dans la monture, voire masqués lorsque les verres sont teintés. En outre, même si les verres ne sont pas teintés, il arrive que la monture masque une partie des points caractéristiques utilisés lors de la détection. Lorsqu'une partie des points caractéristiques est invisible ou leur position à l'image est déformée, le visage détecté, représenté par un modèle, est généralement décalé en position et/ou en orientation par rapport au visage réel, voire à la mauvaise échelle.

Aucun des systèmes actuels ne permet de répondre simultanément à tous les besoins requis, à savoir de proposer une technique de suivi d'un visage portant une paire de lunettes réelle, qui soit plus précis et plus robuste aux mouvements de l'individu, afin d'offrir un rendu amélioré de réalité augmentée.

### EXPOSÉ DE L'INVENTION

La présente invention vise à remédier à tout ou partie des inconvénients de l'état de la technique cités ci-dessus.

À cet effet, l'invention vise, un procédé de suivi d'un visage d'un individu dans un flux vidéo acquis par un dispositif d'acquisition d'images, le visage portant une paire de lunettes, le flux vidéo comprenant une pluralité d'images acquises successivement.

Le procédé de suivi comprend une étape d'évaluation de paramètres d'une représentation du visage comprenant un modèle de la paire de lunettes et un modèle du visage de telle sorte que ladite représentation du visage se superpose à l'image du visage dans le flux vidéo.

Selon l'invention, tout ou partie des paramètres de la représentation sont évalués en tenant compte d'au moins une contrainte de proximité entre au moins un point du modèle du visage et au moins un point du modèle de la paire de lunettes.

A titre d'exemple, une contrainte de proximité peut par exemple définir qu'une branche de la paire de lunettes repose au niveau de la jonction entre le pavillon de l'oreille et le crâne, du côté supérieur, à savoir du côté de l'hélix.

En d'autres termes, la contrainte de proximité est définie entre une zone du modèle du visage et une zone du modèle de la paire de lunettes, la zone pouvant être un point ou un ensemble de points, tel qu'une surface ou une arête.

On entend par proximité une distance nulle ou inférieure à un seuil prédéterminé, par exemple de l'ordre de quelques millimètres.

Ainsi, l'utilisation d'une contrainte de proximité au cours de l'évaluation des paramètres de la représentation du visage permet d'obtenir une pose plus fidèle de la représentation du visage par rapport à la caméra, avec un nombre limité de calculs. Un suivi en temps réel de l'individu peut par conséquent être effectué de manière plus robuste au regard de mouvements inopinés de l'individu par rapport au dispositif d'acquisition d'images.

En outre, l'utilisation conjointe du modèle de la paire de lunettes et du modèle du visage permet d'améliorer la position du visage, notamment par rapport au suivi d'un visage sans lunettes. En effet, dans ce dernier cas, la position des points caractéristiques des tempes est généralement imprécise. Le suivi de la paire de lunettes permet d'apporter une meilleure estimation de la pose de la représentation du visage dans la mesure où les branches de la paire de lunettes se superposant aux tempes de l'individu permettent d'obtenir des informations plus précises sur les points caractéristiques détectés dans une zone de l'image comprenant une tempe de l'individu.

Préférentiellement, les paramètres de la représentation comprennent des valeurs externes à la représentation du visage et des valeurs internes à la représentation du visage, les valeurs externes comprenant une position tridimensionnelle et une orientation tridimensionnelle de la représentation du visage par rapport au dispositif d'acquisition d'images, les valeurs internes comprenant une position tridimensionnelle et une orientation tridimensionnelle du modèle de la paire de lunettes par rapport au modèle du visage, lesdits paramètres étant évalués par rapport à une pluralité de points caractéristiques de ladite représentation du visage, préalablement détectés dans une image du flux vidéo, dite première image, ou dans un jeu d'images acquises simultanément par une pluralité de dispositif d'acquisition d'images, le jeu d'images comprenant ladite première image.

En d'autres termes, la représentation du visage pouvant être appelée avatar comprend des paramètres externes de positionnement et d'orientation dans un environnement tridimensionnel, et des paramètres internes de positionnement et d'orientation relatifs entre le modèle du visage et le modèle de la paire de lunettes. D'autres paramètres internes peuvent être ajoutés tels que les paramètres de configuration de la paire de lunettes : type de monture, taille de monture, matériau, etc. Les paramètres de configuration peuvent également comprendre des paramètres liés à la déformation de la monture de la paire de lunettes et notamment des branches, lorsque la paire de lunettes est portée sur le visage de l'individu. De tels paramètres de configuration peuvent être par exemple les angles d'ouverture ou de fermeture des branches par rapport à un plan de référence tel qu'un plan principal, ou tangent, de la face de la paire de lunettes.

La représentation du visage comprend des modèles tridimensionnels du visage et de la paire de lunettes.

Dans des modes particuliers de mise en œuvre de l'invention, tout ou partie des paramètres de la représentation sont mis à jour par rapport à la position de tout ou partie des points caractéristiques, suivis ou détectés, dans une deuxième image du flux vidéo ou dans une deuxième série d'images acquises simultanément par la pluralité de dispositif d'acquisition d'images, le deuxième jeu d'images comprenant ladite deuxième image.

Ainsi, la mise à jour des paramètres de la représentation, et notamment des valeurs de positionnement et d'orientation relatifs entre le modèle de la paire de lunettes et le modèle du visage, voire des paramètres de configuration, permet d'obtenir un suivi du visage de l'individu plus robuste et plus précis.

Avantageusement, la deuxième image ou le deuxième jeu d'images présente une vue du visage de l'individu selon un angle distinct de celui de la première image ou du premier jeu d'images.

Dans des modes particuliers de mise en œuvre de l'invention, tout ou partie des paramètres de la représentation sont également évalués en tenant compte d'au moins une contrainte de proximité entre un point tridimensionnel d'un des modèles inclus dans la représentation du visage et au moins un point, ou une ligne de niveau, inclus(e) dans au moins une image du flux vidéo.

Dans des modes particuliers de mise en œuvre de l'invention, tout ou partie des paramètres de la représentation sont également évalués en tenant compte d'au moins une contrainte de dimension d'un des modèles inclus dans la représentation du visage.

Dans des modes particuliers de mise en œuvre de l'invention, le procédé comprend une étape d'appariement de deux points distincts appartenant soit à l'un des deux modèles compris dans la représentation du visage, soit chacun à un modèle distinct parmi les modèles compris dans la représentation du visage.

L'appariement de deux points permet notamment de contraindre une relation de distance entre ces deux points, telle qu'une proximité ou une dimension connue entre ces deux points. Une dimension connue est par exemple une distance interpupillaire pour un visage, une largeur d'une monture, une taille caractéristique ou moyen d'un iris, ou toute combinaison de ces valeurs selon une ou des loi(s) de distribution autour d'une valeur moyenne connue de l'un de ces valeurs.

Dans des modes particuliers de mise en œuvre de l'invention, le procédé comprend une étape préalable d'appariement d'un point d'un des deux modèles compris dans la représentation du visage avec au moins un point d'une image acquise par un dispositif d'acquisition d'images.

L'appariement d'un point d'un modèle avec un point d'une image ou un ensemble de points tel qu'une courbe de niveau, est généralement effectué automatiquement.

Dans des modes particuliers de mise en œuvre de l'invention, au cours de l'évaluation des paramètres de la représentation, un alignement du modèle de la paire de lunettes avec une image de la paire de lunettes dans le flux vidéo est effectué consécutivement à un alignement du modèle du visage avec une image du visage dans le flux vidéo.

Dans des modes particuliers de mise en œuvre de l'invention, l'alignement du modèle du visage est effectué en minimisant la distance entre des points caractéristiques du visage détectés dans l'image du visage et des points caractéristiques du modèle du visage projetés dans ladite image.

Dans des modes particuliers de mise en œuvre de l'invention, l'alignement du modèle de la paire de lunettes est effectué en minimisant la distance entre au moins une partie du contour de la paire de lunettes dans l'image et une partie de contour similaire du modèle de la paire de lunettes projeté dans ladite image.

Il convient en effet de souligner que le modèle de la paire de lunettes est un modèle 3D. Une projection de ce modèle 3D est ainsi effectuée dans l'image afin de déterminer un contour similaire qui est utilisé dans le calcul de minimisation de la distance avec le contour de la paire de lunettes détectée dans l'image.

Dans des modes particuliers de mise en œuvre de l'invention, les paramètres de la représentation comprennent également un jeu de paramètres de configuration du modèle du visage et/ou un jeu de paramètres de configuration du modèle de la paire de lunettes.

Les paramètres de configuration du modèle du visage ou ceux du modèle de la paire de lunettes peuvent être par exemple des paramètres morphologiques caractérisant respectivement la forme et la taille du modèle du visage ou celles du modèle de la paire de lunettes. Les paramètres de configuration peuvent également comprendre des paramètres de déformation du modèle, notamment dans le cadre d'une paire de lunettes, pour tenir compte de la déformation d'une branche voire de la face de la paire de lunettes, voire de l'ouverture/fermeture de chaque branche par rapport à la face de la paire de lunettes.

Dans le cadre du modèle de visage, les paramètres de configuration peuvent également comprendre des paramètres d'ouverture et de fermeture des paupières, de la bouche, ou encore des paramètres liés aux déformations de la surface du visage dues aux expressions.

Dans des modes particuliers de mise en œuvre de l'invention, les paramètres de la représentation comprennent tout ou partie de la liste suivante :
- une position tridimensionnelle de la représentation du visage ;
- une orientation tridimensionnelle de la représentation du visage ;
- une taille du modèle de la paire de lunettes ;
- une taille du modèle du visage ;
- une position tridimensionnelle relative entre le modèle de la paire de lunettes et le modèle du visage ;
- une orientation tridimensionnelle relative entre le modèle de la paire de lunettes et le modèle du visage ;
- un ou des paramètre(s) de configuration du modèle de la paire de lunettes ;
- un ou des paramètre(s) de configuration du modèle du visage ;
- un ou des paramètre(s) de la caméra.

Dans des modes particuliers de mise en œuvre de l'invention, le procédé de suivi comprend des étapes de :
- détection d'une pluralité de points du visage dans une première image du flux vidéo ;
- initialisation du jeu de paramètres du modèle du visage par rapport à l'image du visage dans ladite première image initiale ;
- détection d'une pluralité de points d'une paire de lunettes portée par le visage de l'individu dans une deuxième image du flux vidéo, dite deuxième image initiale, la deuxième image initiale étant soit postérieure ou antérieure à la première image initiale dans le flux vidéo, soit identique à la première image dans le flux vidéo ;
- initialisation du jeu de paramètres du modèle de la paire de lunettes par rapport à l'image de la paire de lunettes dans ladite deuxième image initiale.

Dans des modes particuliers de mise en œuvre de l'invention, l'initialisation des paramètres du modèle du visage est effectuée par l'intermédiaire d'une méthode d'apprentissage profond analysant tout ou partie des points détectés du visage.

Dans des modes particuliers de mise en œuvre de l'invention, la méthode d'apprentissage profond détermine également une position initiale du modèle du visage dans le repère tridimensionnel.

Dans des modes particuliers de mise en œuvre de l'invention, le procédé de suivi comprend également une étape de détermination d'une échelle de l'image de la paire de lunettes portée par le visage de l'individu par l'intermédiaire d'une dimension dans l'image d'un élément de taille connue de la paire de lunettes.

Dans des modes particuliers de mise en œuvre de l'invention, la détermination de l'échelle est effectuée par l'intermédiaire d'une reconnaissance préalable de la paire de lunettes portée par le visage de l'individu.

Dans des modes particuliers de mise en œuvre de l'invention, des images acquises par un deuxième dispositif d'acquisition d'images sont utilisées pour évaluer les paramètres de la représentation.

Dans des modes particuliers de mise en œuvre de l'invention le modèle de la paire de lunettes de la représentation correspond à une modélisation préalable de ladite paire de lunettes, et varie uniquement en déformation.

La forme et la taille du modèle de la paire de lunettes restant invariant, cela permet d'obtenir une meilleure résolution dans un temps de calcul plus court.

L'invention vise également un procédé de réalité augmentée comprenant des étapes de :
- acquisition d'au moins un flux d'images d'un individu portant une paire de lunettes sur son visage par au moins un dispositif d'acquisition d'images ;
- suivi du visage de l'individu par un procédé de suivi selon l'un quelconque des modes de mise en œuvre précédents, une position et une orientation d'une représentation du visage ;
- modification de tout ou partie des images dudit ou d'un desdits flux d'images, appelé flux vidéo principal, acquis par le dispositif d'acquisition d'images ou par l'un des dispositifs d'acquisition d'images, appelé dispositif principal d'acquisition d'images, grâce à la représentation du visage se superposant en temps réel au visage de l'individu sur le flux vidéo principal ;
- affichage sur un écran du flux vidéo principal modifié précédemment.

Il convient de souligner que les étapes du procédé de réalité augmentée sont avantageusement effectuées en temps réel.

L'invention vise également un dispositif électronique comportant une mémoire informatique stockant des instructions d'un procédé de suivi ou de réalité augmentée selon l'un quelconque des modes de mise en œuvre précédents.

Avantageusement, le dispositif électronique comprend un processeur apte à traiter des instructions dudit procédé.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
- la figure 1 est une vue schématique d'un dispositif de réalité augmentée mettant en œuvre un mode de mise en œuvre du procédé de détection et de suivi selon l'invention ;
- la figure 2 est un schéma synoptique du procédé de détection et de suivi mis en œuvre par le dispositif de réalité augmentée de la figure 1 ;
- la figure 3 représente une vue du masque d'une paire de lunettes (sous-figure a) et de la répartition des points du contour du masque selon des catégories (sous-figures b et c) ;
- la figure 4 est une vue en perspective de la face d'un modèle de paire de lunettes, avec et sans enveloppe extérieure (respectivement sous figure b et a) ;
- la figure 5 illustre l'étape de régression du procédé de la figure 2 à l'aide d'un extrait d'une image acquise par le dispositif d'acquisition d'images du dispositif de la figure 1, sur laquelle est superposée un modèle d'une paire de lunettes ;
- la figure 6 illustre les contraintes de positionnement entre un modèle de la paire de lunettes et un modèle du visage ;
- la figure 7 est une vue en perspective d'un modèle paramétrique (3DMM) d'une paire de lunettes ;
- la figure 8 est une vue simplifiée de la face du modèle paramétrique de la figure 7.

### DESCRIPTION DÉTAILLÉE DE MODES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

### Exemple d'un mode de réalisation particulier

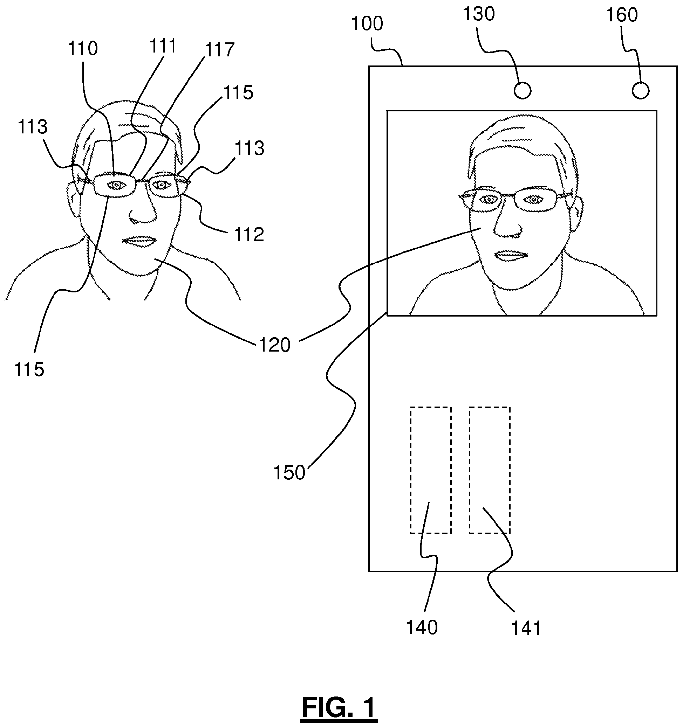

La figure 1 représente un dispositif 100 de réalité augmentée utilisé par un individu 120 portant une paire de lunettes 110 sur son visage 125. La paire de lunettes 110 comprend usuellement une monture 111 comportant une face 112 et deux branches 113 s'étendant de part et d'autre du visage de l'individu 120. En outre, la face 112 permet notamment de porter des verres 114 placés à l'intérieur des deux cercles 115 configurés dans la face 112. Deux plaquettes (non représentées sur la figure 1) sont solidarisées chacune en saillie sur le bord d'un cercle 115 distinct de telle sorte qu'elles puissent reposer sur le nez 121 de l'individu 120. Un pont 117 reliant les deux cercles 115 chevauchent le nez 121 lorsque la paire de lunettes 110 est portée par le visage de l'individu 120.

Le dispositif 100 comprend un dispositif principal d'acquisition d'images, en l'occurrence une caméra 130, acquérant une pluralité d'images successives formant un flux vidéo, s'affichant en temps réel sur un écran 150 du dispositif 100. Un processeur informatique 140 inclus dans le dispositif 100 traite en temps réel les images acquises par la caméra 130 selon les instructions d'un procédé suivi selon l'invention qui sont stockées dans une mémoire informatique 141 du dispositif 100.

Optionnellement, le dispositif 100 peut également comprendre au moins un dispositif secondaire d'acquisition d'images, en l'occurrence au moins une caméra secondaire 160, pouvant être orienté similairement ou différemment par rapport à la caméra 130, permettant d'acquérir un deuxième flux d'images du visage 125 de l'individu 120. Auquel cas, il convient de souligner que la position et l'orientation relative de la caméra secondaire 160, ou de chaque caméra secondaire, par rapport à la caméra 130 sont généralement avantageusement connues.

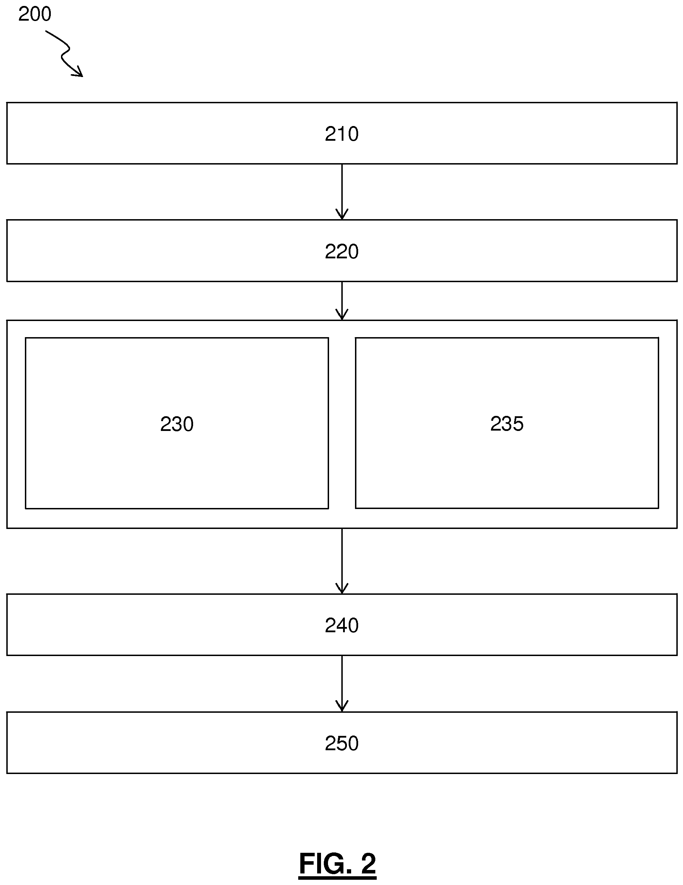

La figure 2 illustre sous la forme d'un schéma synoptique le procédé 200 de suivi dans le flux vidéo acquis par la caméra 130 du visage de l'individu 120.

Tout d'abord, il convient de souligner que le procédé 200 de suivi est généralement mis en œuvre en boucle sur des images, généralement successives, du flux vidéo. Pour chaque image, plusieurs itérations de chaque étape peuvent être effectuées notamment pour la convergence des algorithmes utilisés.

Le procédé 200 comprend une première étape 210 de détection de la présence du visage de l'individu 120 portant la paire de lunettes 110 dans une image du flux vidéo, dite image initiale.

Cette détection peut être effectuée de plusieurs manières :
- soit à partir d'une base d'apprentissage de visages portant une paire de lunette en utilisant un algorithme d'apprentissage profond, également connu sous le terme anglais « *deep learning »,* préalablement entraîné sur une base de données comprenant des images de visage portant une paire de lunettes ;
- soit en utilisant un modèle tridimensionnel d'un visage portant une paire de lunettes que l'on cherche à faire correspondre à l'image du visage dans l'image initiale en déterminant une pose, en orientation et en dimension, du modèle tridimensionnel par rapport à la caméra 130. La correspondance du modèle du visage et de l'image du visage dans l'image initiale peut être notamment effectuée par l'intermédiaire d'une projection sur l'image initiale du modèle du visage portant une paire de lunettes. Il convient de souligner que cette correspondance peut s'effectuer même si une partie du visage ou de la paire de lunettes est cachée dans l'image, comme c'est le cas par exemple lorsque le visage est tourné par rapport à la caméra ou lorsque des éléments viennent se superposer au visage comme une paire de lunettes ou des cheveux, ou se superposer à la paire de lunettes comme des cheveux.

Alternativement, l'étape 210 de détection dans l'image initiale du visage de l'individu 120 portant une paire de lunettes 110 peut être effectuée en détectant dans un premier temps l'un des deux éléments, par exemple le visage, puis dans un deuxième temps l'autre élément, à savoir ici la paire de lunettes. La détection du visage s'effectue par exemple par l'intermédiaire de la détection de points caractéristiques du visage dans l'image. Une telle méthode de détection du visage est connue de l'homme du métier. La détection de la paire de lunettes peut s'effectuer par exemple par l'intermédiaire d'un algorithme d'apprentissage profond, également connu sous le terme anglais « *deep learning »,* préalablement entraîné sur une base de données d'images de paire de lunettes, préférentiellement portée par un visage.

Il convient de souligner que l'étape 210 de détection peut n'être effectuée qu'une seule fois pour une pluralité d'images du flux vidéo.

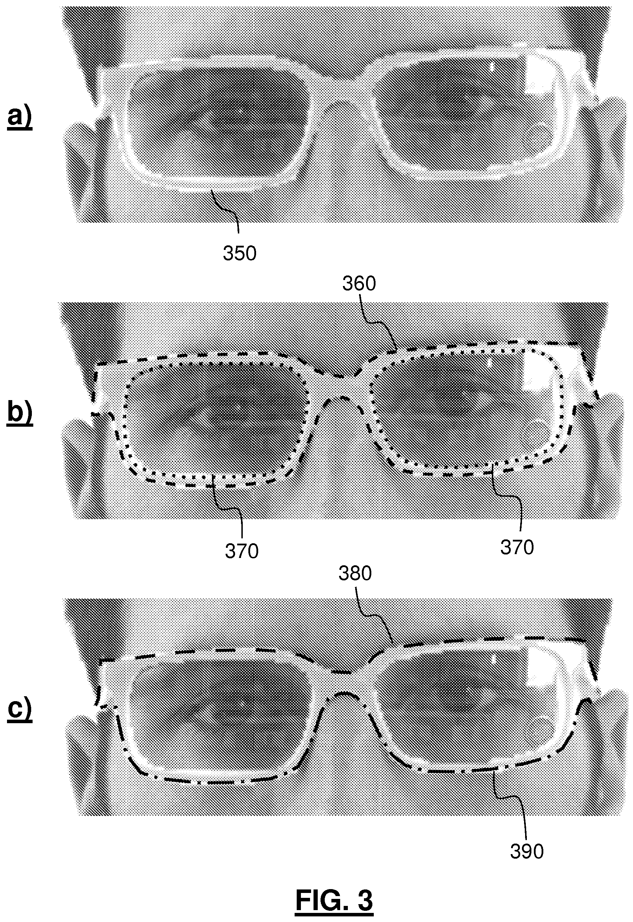

Comme illustré sur la figure 3, l'algorithme d'apprentissage permet notamment de calculer un masque 350 binaire de la paire de lunettes pour chacune des images acquises.

Les points contour du masque, notés p2D, sont associés chacun à au moins une catégorie telle que :
- un contour extérieur 360 du masque ;
- un contour intérieur 370 du masque, correspondant généralement à un contour d'un verre ;
- un contour 380 du haut du masque ;
- un contour 390 du bas du masque.

Alternativement, les points contour du masque, p2D, sont calculés en utilisant une distance robuste, c'est-à-dire variant peu entre deux itérations successives, entre des points caractéristiques de la paire de lunettes détectés dans l'image et des points du contour du masque.

Après avoir détecté le visage de l'individu 120 portant la paire de lunettes 110, le procédé 200 comprend une deuxième étape 220 d'alignement d'une représentation du visage de l'individu, appelée par la suite « avatar », avec l'image du visage de l'individu 120 dans l'image initiale. L'avatar comprend ici avantageusement deux modèles paramétriques, l'un correspondant à un modèle de visage sans paire de lunettes et l'autre à un modèle d'une paire de lunettes. Il convient de souligner que les modèles paramétriques sont généralement placés dans un espace virtuel dont l'origine du repère correspond à la caméra 130. On parlera ainsi de repère de la caméra.

L'utilisation conjointe de ces deux modèles paramétriques permet d'accroître la performance de la régression et d'obtenir une meilleure estimation de la position du modèle du visage de l'individu par rapport à la caméra.

En outre, les deux modèles paramétriques de l'avatar sont ici avantageusement liés entre eux par des paramètres relatifs d'orientation et de positionnement. Initialement, les paramètres relatifs d'orientation et de positionnement correspondent par exemple à une pose standard du modèle paramétrique de la paire de lunettes par rapport au modèle paramétrique du visage, c'est-à-dire de telle sorte que la monture repose sur le nez, en face des yeux de l'individu et que les branches s'étendant le long des tempes de l'individu reposent sur les oreilles de ce dernier. Cette pose standard est par exemple calculée par un positionnement moyen d'une paire de lunettes positionné naturellement sur le visage d'un individu. Il convient de souligner que la paire de lunettes peut être plus ou moins avancée sur le nez en fonction des individus.

Le modèle paramétrique de la paire de lunettes est dans le présent exemple non limitatif de l'invention un modèle comportant une monture tridimensionnelle dont l'enveloppe comporte une épaisseur non nulle en au moins une section. Avantageusement, l'épaisseur est non nulle en chaque partie de la section de la monture.

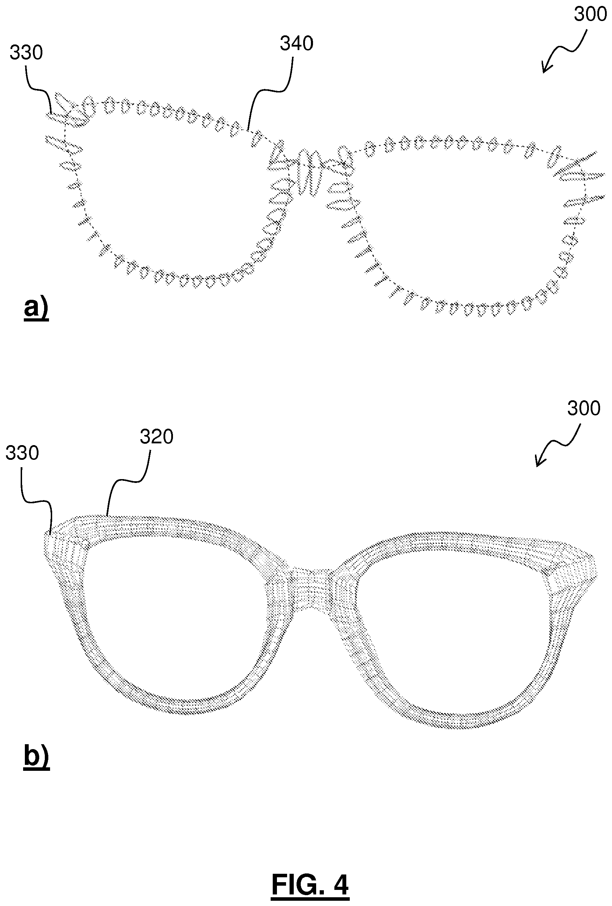

La figure 4 présente la face 300 du modèle paramétrique de la paire de lunettes selon deux vues. La première vue, notée 4a, correspond à une vue du squelette de la face 300, sans enveloppe extérieure. La deuxième vue, notée 4b, correspond à la même vue mais avec l'enveloppe extérieure 320. Comme illustré, le modèle paramétrique de la paire de lunettes peut être représenté par une succession de contours 330 de section chacune perpendiculaire à une âme 340 de la monture de la paire de lunettes. Les contours 330 forment ainsi un squelette pour l'enveloppe extérieure 320. Ce modèle paramétrique est de type 3D avec épaisseur.

Il convient de souligner que le modèle paramétrique de la paire de lunettes peut avantageusement comprendre un nombre prédéterminé de sections numérotées de telles sortes que la position des sections autour de la monture est identique pour deux modèles distincts de paire de lunettes. La section correspondant au point de la monture, tel qu'un point bas d'un cercle, un point haut d'un cercle, un point de jonction entre un cercle et le pont, ou un point de jonction entre un cercle et un tenon portant une charnière avec une branche, a ainsi le même numéro dans les deux modèles distincts. Il est ainsi plus facile d'adapter le modèle de la paire de lunettes aux indications de dimensions de la monture. Ces indications, couramment appelées par le terme anglais « *frame marking »,* définissent la largeur d'un verre, celle du pont ou encore la longueur des branches. Ces informations peuvent alors servir dans la définition de contraintes entre deux points, correspondant par exemple au centre ou au bord de deux sections choisies en fonction de leur position sur la monture. Le modèle de la paire de lunettes peut ainsi être modifié tout en respectant les contraintes de dimensions.

Un exemple de modèle paramétrique d'une paire de lunettes utilisé par le présent procédé est présenté ultérieurement plus en détails dans une section intitulée « Exemple d'un modèle paramétrique d'une paire de lunettes ».

Dans des modes alternatifs de mise en œuvre de l'invention, le modèle paramétrique de la paire de lunettes comporte une monture tridimensionnelle d'épaisseur nulle. Il s'agit alors d'un modèle de type 3D sans épaisseur.

L'ensemble des paramètres permettant de définir la morphologie et la taille de la paire de lunettes sont appelés paramètres de configuration.

Il convient de souligner que la forme initiale de la monture du modèle paramétrique peut avantageusement correspondre à la forme de la monture de la paire de lunettes qui a été préalablement modélisée par une méthode telle que décrite par exemple dans le brevet français publié sous le numéro FR 2955409 ou dans la demande internationale de brevet publiée sous le numéro WO 2013/139814.

Le modèle paramétrique de la paire de lunettes peut également être avantageusement déformé, par exemple au niveau des branches ou de la face, qui sont généralement formés dans un matériau pouvant se déformer de manière élastique. Les paramètres de déformation sont inclus dans les paramètres de configuration du modèle de paire de lunettes. Dans le cas où le modèle de la paire de lunettes est connu, grâce par exemple une modélisation préalable de la paire de lunettes 110, le modèle de la paire de lunettes peut avantageusement rester invariant en taille et en forme au cours de la résolution. Seule la déformation du modèle de la paire de lunettes est alors calculée. Le nombre de paramètres à calculer étant réduit, le temps de calcul est plus court pour obtenir un résultat satisfaisant.

Pour aligner les deux modèles paramétriques de la représentation du visage par rapport à l'image de la paire de lunettes et du visage dans l'image initiale, une régression des points des modèles paramétriques est effectuée au cours de la deuxième étape 220 afin que les modèles paramétriques correspondent en forme, en taille, en position et en orientation respectivement à la paire de lunettes 110 portée par l'individu 120 et au visage de l'individu 120.

Les paramètres de l'avatar traités par la régression sont ainsi dans le présent exemple non limitatif de l'invention :
- la position tridimensionnelle de l'avatar, c'est-à-dire de l'ensemble {modèle de paire de lunettes, modèle de visage} ;
- l'orientation tridimensionnelle de l'avatar ;
- la taille du modèle de la paire de lunettes ;
- la taille du modèle du visage ;
- la position tridimensionnelle relative entre le modèle de la paire de lunettes et le modèle du visage ;
- l'orientation tridimensionnelle relative entre le modèle de la paire de lunettes et le modèle du visage ;
- optionnellement, des paramètres de configuration du modèle de la paire de lunettes ;
- optionnellement, des paramètres de configuration du modèle du visage tels que des paramètres morphologiques permettant de définir la forme, la taille, la position des différents éléments constitutifs d'un visage tel que notamment le nez, la bouche, les yeux, les tempes, les joues, etc . Les paramètres de configuration peuvent également comprendre des paramètres d'ouverture ou de fermeture des paupières ou de la bouche, et/ou des paramètres liés aux déformations de la surface du visage dues aux expressions ;
- optionnellement des paramètres de la caméra, tels qu'une focale ou un paramètre de calibration métrique.

Alternativement, seule une partie des paramètres de l'avatar listés précédemment sont traités par la régression.

Les paramètres de la caméra peuvent avantageusement être calculés lorsque la géométrie 3D du modèle de la paire de lunettes est connue, par exemple lorsque la paire de lunettes 110 portée par l'individu 120 a été reconnue. L'ajustement des paramètres de la caméra contribue à l'obtention d'une meilleure estimation des paramètres de l'avatar, et par conséquent à un meilleur suivi du visage dans l'image.

La régression est effectuée avantageusement ici en deux temps. Dans un premier temps, une minimisation des points caractéristiques du modèle du visage avec les points caractéristiques détectés sur l'image initiale est effectuée pour obtenir une position estimative de l'avatar dans le repère de la caméra.

Dans un deuxième temps, les paramètres de l'avatar sont affinés en effectuant une régression des points du contour du modèle de la paire de lunettes par rapport à la paire de lunettes telle que visible sur l'image initiale du flux vidéo. Les points du contour du modèle de la paire de lunettes considérés lors de la régression sont généralement issus de la monture de la paire de lunettes.

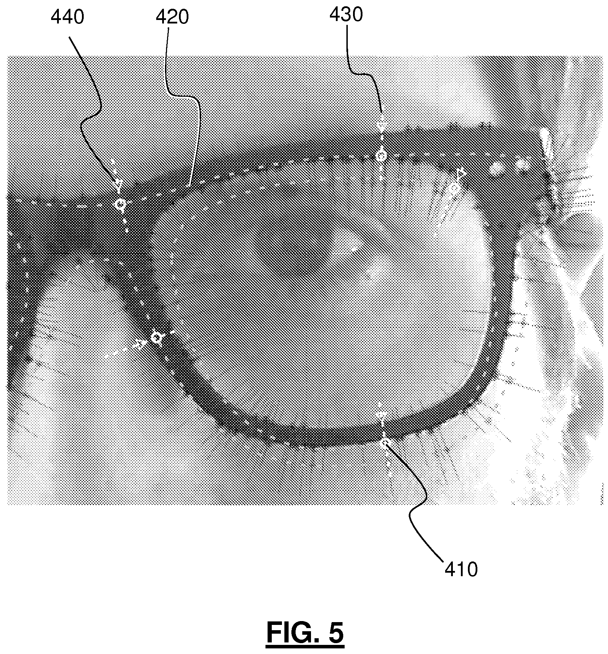

A cet effet, comme illustré en figure 5, les points 410 considérés du contour du modèle 420 de la paire de lunettes sont ceux dont les normales 430 sont perpendiculaires à l'axe entre le point 410 correspondant et la caméra. A chaque point 410 considéré du contour du modèle de la paire de lunettes est associé un point du contour de la paire de lunettes sur l'image initiale, en recherchant le point 440 le long de la normale 430 ayant le plus fort gradient, par exemple dans un spectre de couleur donné tel qu'en niveau de gris. Le contour de la paire de lunettes peut également être déterminé par l'intermédiaire d'une méthode d'apprentissage profond, également connue sous le terme anglais « *deep learning »,* préalablement entraînée sur des images de paires de lunettes segmentées, préférentiellement portées par un visage. En minimisant la position entre les points des contours du modèle et de la paire de lunettes sur l'image initiale, il est ainsi possible d'affiner les paramètres de l'avatar dans le repère de la caméra.

Il convient de souligner que par soucis de clarté, seuls cinq points 410 ont été mis en avant sur la figure 5. Le nombre de points utilisés par la régression est généralement nettement plus élevé. Les points 410 sont représentés par un cercle sur la figure 4, les points 440 correspondent à un sommet d'un triangle glissant le long d'une normale 430.

L'association d'un point du contour du modèle de la paire de lunettes avec un point du contour de la paire de lunettes 110 dans l'image correspond à un appariement d'un point 3D du modèle de la paire de lunettes avec un point 2D de l'image. Il convient de souligner que cet appariement est préférentiellement évalué à chaque itération, voire à chaque image, car le point correspondant dans l'image peut avoir glissé d'une image à l'autre.

En outre, la ou les catégories du point du contour dans l'image étant avantageusement connue(s), l'appariement de ce point avec un point 3D du modèle de la paire de lunettes peut être effectué de manière plus efficace en appariant des points ayant les mêmes catégories. Il convient en effet de souligner que les points du modèle de la paire de lunettes peuvent également être classés selon les mêmes catégories que les points du contour du masque de la paire de lunettes dans l'image.

Afin d'améliorer la régression autour du positionnement du modèle de la paire de lunettes, un contour d'une section est avantageusement associé à la majorité des points considérés du contour du modèle de la paire de lunettes. La section associée à un point correspond généralement à la tranche de la monture comprenant ce point. Chaque section est définie par un polygone comprenant un nombre prédéterminé d'arrêtes. Ainsi, lors de la régression, le calcul de la normale est amélioré en étant plus précis, ce qui permet d'avoir une meilleure estimation de la pose du modèle de la paire de lunettes par rapport à l'image. Cette amélioration est notamment applicable dans le cas de l'utilisation d'un modèle paramétrique de la paire de lunettes 3D avec épaisseur.

Il convient également de souligner que lors de la régression, des contraintes de positionnement entre le modèle du visage et le modèle de la paire de lunettes sont avantageusement prises en compte afin de réduire le temps de calcul tout en offrant une meilleure qualité de pose. Les contraintes indiquent par exemple une collision de points entre une partie du modèle du visage et une partie du modèle de la paire de lunettes. Ces contraintes traduisent par exemple que les cercles, via les plaquettes ou non, de la paire de lunettes reposent sur le nez et que les branches reposent sur les oreilles. Généralement les contraintes de positionnement entre le modèle du visage et le modèle de la paire de lunettes permettent de paramétrer le positionnement de la paire de lunettes sur le visage avec un seul paramètre, par exemple la position de la paire de lunettes sur le nez de l'individu. Entre deux positions sur le nez, la paire de lunettes effectue une translation selon une courbe 3D correspondant à l'arête du nez, voire une rotation selon un axe perpendiculaire à ce plan de symétrie médian. Localement entre deux points proches, il peut être considéré que la translation de la paire de lunettes selon la courbe 3D suit un plan de symétrie locale du nez.

En d'autres termes, la contrainte est traduite par un appariement d'un point du modèle du visage avec un point du modèle de la paire de lunettes. Il convient de souligner que l'appariement entre les deux points peut être de type partiel, à savoir ne porter que sur un type de coordonnées, par exemple seulement l'axe des x afin de laisser libre la translation d'un des deux modèles par rapport à l'autre selon les deux autres axes.

Par ailleurs, chacun des deux modèles paramétriques compris dans l'avatar, c'est-à-dire celui du visage et celui de la paire de lunettes, peut également être avantageusement contraints selon une dimension connue telle qu'une distance interpupillaire préalablement mesurée pour le visage ou une dimension caractéristique de la monture préalablement reconnue. Un appariement entre deux points du même modèle peut ainsi être effectué pour contraindre la distance entre ces deux points selon la dimension connue.

Pour plus de détails mathématiques de l'algorithme, il est possible de se référer à la présentation effectuée ultérieurement au cours de la section intitulée « Détails du procédé mis en œuvre ».

Il convient de souligner que lorsqu'au moins une caméra secondaire est disponible, plusieurs vues du visage de l'individu portant la paire de lunettes sont disponibles, ce qui permet d'améliorer le calcul de régression des paramètres de l'avatar. En effet, les différentes vues sont acquises avec un angle distinct, permettant ainsi d'améliorer la connaissance du visage de l'individu en affichant des parties cachées sur l'image acquise par la caméra principale.

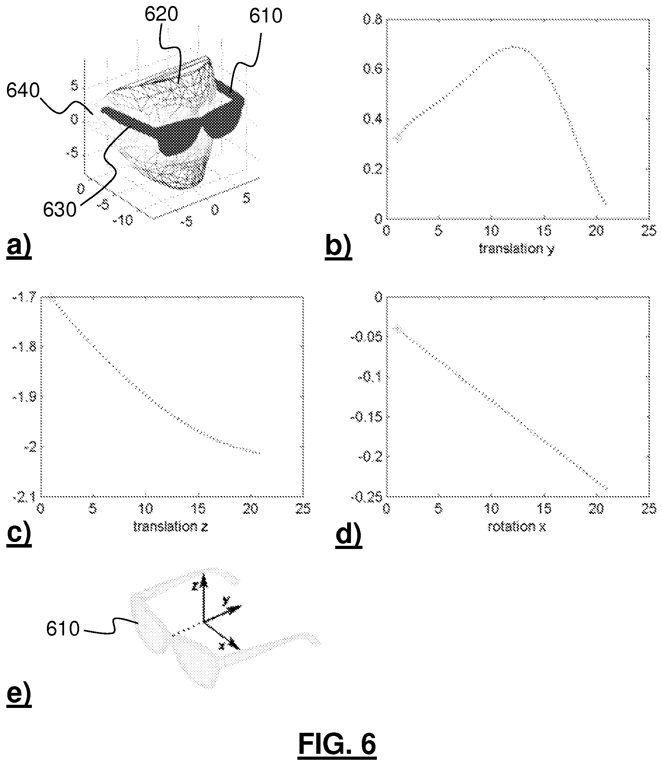

La figure 6 illustre le positionnement du modèle paramétrique 610 de la paire de lunettes sur le modèle paramétrique 620 du visage de l'avatar qui est visible selon une vue en perspective en sous-figure a. Le repère utilisé est illustré par la sous-figure e de la figure 6. Le déplacement du modèle paramétrique 610 de la paire de lunettes est ici paramétré selon un déplacement des branches 630 sur les oreilles 640, correspondant à la translation selon l'axe z (sous-figure c de la figure 6). La translation selon l'axe y correspondante est visible sur la sous-figure b de la figure 6. La rotation autour de l'axe x est illustré sur la sous-figure d de la figure 6.

Des contraintes de non collision entre certaines parties du modèle du visage et certaines parties du modèle de la paire de lunettes peuvent également être ajoutées afin d'éviter un mauvais positionnement du modèle de la paire de lunettes sur le modèle du visage, par exemple une branche dans un œil de l'individu, etc.

Une difficulté surmontée par la présente invention est la gestion des parties cachées de la paire de lunettes dans l'image initiale, qui peuvent entraîner des erreurs dans la régression du modèle paramétrique de la paire de lunettes, notamment au niveau de la position et de l'orientation du modèle paramétrique par rapport à la paire de lunettes 110 réellement portée par l'individu 120. Ces parties cachées correspondent généralement à des parties de la monture qui sont masquées soit par le visage de l'individu, par exemple quand le visage est tourné par rapport à la caméra afin de voir un profil du visage, soit directement par la paire de lunettes, par exemple par des verres teintés. Il convient en outre de souligner que la partie des branches venant se poser sur chaque oreille est généralement occultée, quel que soit l'orientation du visage de l'individu 120, par une oreille et/ou par des cheveux de l'individu 120.

Ces parties cachées peuvent être par exemple estimées lors de la détection en considérant un modèle de segmentation de la monture et/ou des points du contour de ces parties cachées. Les parties cachées de la paire de lunettes peuvent également être estimées en calculant une pose d'un modèle paramétrique d'une paire de lunettes par rapport à la position estimée du visage de l'individu 120. Le modèle paramètre utilisé ici peut être le même que celui utilisé pour l'avatar.

L'alignement du modèle paramétrique de la paire de lunettes permet également de reconnaître le modèle de la paire de lunettes 110 réellement portée par l'individu 120. En effet, la régression des points permet d'obtenir un contour approximatif 3D d'au moins une partie de la paire de lunettes 110. Ce contour approximatif est ensuite comparé aux contours de paires de lunettes préalablement modélisées, enregistrés dans une base de données. L'image incluse dans le contour peut également être comparé à l'apparence des paires de lunettes enregistrées dans la base de données pour une meilleure reconnaissance du modèle de la paire de lunettes 110 portée par l'individu 120. Il convient en effet de souligner que les modèles de paires de lunettes stockées dans la base de données ont généralement également été modélisés en texture et en matière.

Le modèle paramétrique de la paire de lunettes peut être déformé et/ou articulé afin de correspondre au mieux à la paire de lunettes 110 portée par l'individu 120. Généralement, les branches du modèle de la paire de lunettes forment initialement entre elles un angle de l'ordre de 5°. Cet angle peut être ajusté en modélisant la déformation de la paire de lunettes en fonction de la forme de la monture et de la rigidité de la matière utilisée pour les branches, voire également de la matière utilisée pour la face de la monture de la paire de lunettes qui peut être distincte de celle des branches. Une approche paramétrique peut être utilisée pour modéliser la déformation du modèle paramétrique de la paire de lunettes.

Un suivi en temps réel du visage et/ou de la paire de lunettes dans le flux vidéo, sur des images successives à ladite image initiale, est effectué au cours d'une troisième étape 230 du procédé 200 illustré en figure 2.

Le suivi en temps réel peut par exemple être basé sur le suivi de points caractéristiques dans des images successives du flux vidéo, par exemple en utilisant une méthode de flot optique.

Ce suivi peut notamment être effectué en temps réel car la mise à jour des paramètres pour une image du flux vidéo est généralement effectué par rapport aux paramètres d'alignement calculées à l'image précédente.

Afin d'améliorer la robustesse du suivi, l'utilisation d'images-clés, couramment appelées par le terme anglais « keyframe », où la pose de l'avatar par rapport au visage de l'individu est considérée comme satisfaisante peut être utilisée pour apporter des contraintes sur les images présentant des vues du visage orienté de manière similaire au visage dans une image-clé. En d'autres termes, une image-clé d'une sélection d'images du flux vidéo, pouvant également être appelée image de référence, correspond généralement à une des images de la sélection où le score associé à la pose de l'avatar par rapport au visage de l'individu est le plus important. Un tel suivi est par exemple décrit en détails dans la demande internationale de brevet publiée sous le numéro WO 2016/135078.

Il convient de souligner que le choix d'une image-clé peut être effectué de manière dynamique et que la sélection d'images peut correspondre à une séquence continue du flux vidéo.

En outre, le suivi peut utiliser avantageusement plusieurs images-clés, chacune correspondant à une orientation distincte du visage de l'individu.

Il convient également de souligner que le suivi conjoint du visage et de la paire de lunettes permet d'obtenir de meilleurs résultats, plus robustes, car basés sur un nombre de points caractéristiques plus élevés. En outre, les contraintes de positionnement relatif des modèles paramétriques du visage et de la paire de lunettes sont généralement utilisées au cours du suivi, ce qui permet d'obtenir un suivi plus précis de la tête de l'individu en temps réel, et par conséquent une meilleure pose de l'avatar.

Par ailleurs, le suivi d'une paire de lunettes, qui est un objet manufacturé, est généralement plus précis que le suivi d'un visage seul, car la paire de lunettes comporte des amers bien identifiables dans une image, tels qu'une arrête d'une branche, une arrête de la face ou un cercle de la face de la monture.

Il convient de souligner qu'un suivi de la paire de lunettes, sans utilisation d'un modèle paramétrique de la paire de lunettes, serait moins robuste et nécessiterait un grand nombre de calculs pour chaque image. Un tel suivi est ainsi plus difficile à mettre en œuvre en temps réel au regard des puissances de calcul actuellement disponible. Toutefois, compte-tenu de l'augmentation régulière de la puissance des processeurs, un suivi sans utilisation d'un modèle paramétrique de la paire de lunettes pourrait être envisagé lorsque les puissances des processeurs seront suffisantes pour une telle application.

Il convient également de souligner qu'il est possible d'effectuer un suivi de l'individu en se basant uniquement sur le modèle paramétrique de la paire de lunettes. L'optimisation de la pose du modèle de la paire de lunettes par rapport à la caméra, c'est-à-dire de l'alignement du modèle de la paire de lunettes par rapport à l'image, est effectuée pour chaque image.

Une mise à jour des paramètres d'alignement des modèles paramétriques du visage et de la paire de lunettes avec l'image est ensuite effectuée pour chaque nouvelle image du flux vidéo acquis par la caméra 130, concomitamment à l'étape 230 de suivi, au cours d'une étape 235.

Alternativement, la mise à jour des paramètres d'alignement des modèles paramétriques du visage et de la paire de lunettes est effectuée à chaque image-clé.

Cette mise à jour des paramètres d'alignement peut également comprendre le paramètre de pose du modèle paramétrique de la paire de lunettes sur le modèle paramétrique du visage, afin d'améliorer l'estimation du positionnement du visage de l'individu par rapport à la caméra. Cette mise à jour peut notamment être effectuée lorsque le visage de l'individu est orienté différemment par rapport à la caméra, offrant ainsi un autre angle de vue de son visage.

Un raffinement des modèles paramétriques peut être effectué au cours d'une quatrième étape 240 du procédé 200 en analysant les images-clés de référence utilisées au cours du suivi. Ce raffinement permet par exemple de compléter le modèle paramétrique de la paire de lunettes avec des détails de la paire de lunettes 110 qui n'auraient pas été capturés précédemment. Ces détails sont par exemple un relief, une lumière ou une sérigraphie spécifique à la paire de lunettes.

L'analyse des images-clés est effectuée par une méthode d'ajustement de faisceaux, également connue sous le terme anglais de *« bundle adjustment »,* qui permet de raffiner les coordonnées 3D d'un modèle géométrique décrivant un objet de la scène, tel que la paire de lunettes ou le visage. La méthode de *« bundle adjustment »* est basée sur une minimisation des erreurs de reprojection entre les points observés et les points du modèle.

Ainsi, il est possible d'obtenir des modèles paramétriques plus conformes au visage de l'individu portant la paire de lunettes.

L'analyse par la méthode de *« bundle adjustment »* met ici en œuvre des points caractéristiques du visage et des points des lunettes qui sont identifiables avec plus de précision dans l'image-clé. Ces points peuvent être des points du contour du visage ou des lunettes.

Il convient de souligner que la méthode de « *bundle adjustment »* traite de manière générale une scène définie par une série de points 3D pouvant bouger entre deux images. La méthode de *« bundle adjustment »* permet de résoudre simultanément la position tridimensionnelle de chaque point 3D de la scène dans un référentiel donné (par exemple celui de la scène), les paramètres de mouvements relatifs de la scène par rapport à la caméra et les paramètres optiques de la ou des caméra(s) ayant acquises les images.

Des points glissants calculés grâce à une méthode de flot optique, par exemple liés aux points du contour du visage ou des lunettes, peuvent également être utilisés par la méthode de « *bundle adjustment ».* Toutefois, le flot optique se calculant entre deux images distinctes, généralement consécutives dans le flux vidéo, ou entre deux images-clés, la matrice obtenue au cours de la méthode de *« bundle adjustment »* pour les points issus du flot optique est généralement creuse. Pour compenser ce manque d'informations, des points du contour des lunettes peuvent être avantageusement utilisés par la méthode de « *bundle adjustment ».*

Il convient de souligner que de nouvelles informations permettant d'améliorer le modèle paramétrique du visage ou le modèle paramétrique de la paire de lunettes peuvent être obtenues pour une nouvelle image-clé. En outre, une nouvelle détection du visage portant la paire de lunettes, comme celle décrite dans l'étape 210, peut être réalisée dans cette nouvelle image-clé, afin de compléter ou de remplacer les points utilisés par la méthode de *« bundle adjustment ».* Une contrainte de résolution avec un poids plus important peut être associé aux nouveaux points détectés afin d'obtenir que le raffinement des modèles paramétriques soit plus proche de l'image en cours du flux vidéo.

Des points glissants du contour des lunettes peuvent être appariés au modèle 3D de la paire de lunettes sur une ligne de niveau du contour des lunettes, correspondant à l'ensemble des points du modèle de la paire de lunettes dont la normale est à 90 degrés.

Dans un exemple de mise en œuvre de l'invention, les images-clefs correspondent à des images lorsque le visage de l'individu 120 portant la paire de lunettes 110 est de face, et/ou à des images où le visage de l'individu 120 est tourné à gauche ou à droite par rapport au port naturel de la tête d'un angle de l'ordre de 15 degrés par rapport au plan sagittal. Pour ces images-clefs, de nouvelles parties du visage 125 et de la paire de lunettes 110 sont visibles. Les paramètres des modèles du visage et de la paire de lunettes peuvent ainsi être déterminés avec plus de précision. Le nombre d'images-clefs peut être fixé arbitrairement à un nombre compris entre 3 et 5 images afin d'obtenir des résultats satisfaisants dans l'apprentissage du visage 125 et de la paire de lunettes 110 pour établir les modèles correspondants.

La taille de la paire de lunettes 110 portée par l'individu 120 peut également être introduit au cours du procédé 200 lors d'une étape 250, notamment pour obtenir une métrique de la scène, et définir une échelle notamment pour déterminer une mesure optique du visage de l'individu, telle que par exemple une distance interpupillaire ou une taille d'un iris qui peut être définie comme une taille moyenne.

La taille de la paire de lunettes 110 peut être définie de manière statistique par rapport à une liste de paires de lunettes préalablement définie, ou correspondre à la taille effective de la paire de lunettes 110.

Il peut être prévu une interface pour indiquer au procédé 200 quel est le *« frame marking »* indiqué dans la paire de lunettes 110. Alternativement, une lecture automatique sur une image peut être effectuée par le procédé 200 pour reconnaitre les caractères du « *frame marking »* et obtenir automatiquement les valeurs associées.

Il convient de souligner que lorsque le *« frame marking »* est connu, le modèle paramétrique de la paire de lunettes 110 peut être avantageusement connu, notamment si la paire de lunettes 110 a préalablement été modélisée.

Lorsqu'aucune information de taille sur la paire de lunettes n'est disponible, par exemple lorsque le *« frame marking »* est inconnu, le modèle paramétrique de la paire de lunettes utilisé initialement est un modèle paramétrique standard comprenant des valeurs statistiquement moyennes des paires de lunettes couramment utilisées par les individus. Ce cadre statistique permet d'obtenir un résultat satisfaisant, proche du modèle de la paire de lunettes 110 réellement porté par l'individu 120, chaque nouvelle image améliorant les paramètres du modèle de la paire de lunettes.

Une caméra de profondeur peut également être utilisée au cours du procédé 200 afin d'affiner la forme et la position du visage.

Il convient de souligner que la caméra de profondeur est un type de capteur de profondeur, couramment connu sous le nom anglais de « *depth sensor ».* En outre, la caméra de profondeur, fonctionnant généralement en utilisant l'émission d'une lumière infrarouge, n'est pas suffisamment précise pour acquérir les contours de la paire de lunettes 110 portée par l'individu 120, notamment à cause des problèmes de réfraction, de transmission et/ou de réflexion introduits par les verres et/ou le matériau d'une face de la paire de lunettes. Dans certains cas, des conditions lumineuses, telle que la présence d'une source lumineuse intense dans le champ de la caméra, empêchent le bon fonctionnement de la caméra de profondeur à infrarouge en introduisant un bruit important empêchant toutes mesures fiables. Cependant, les mesures de profondeur peuvent être utilisées sur des parties visibles du visage, afin de garantir des mesures de profondeur sur la surface visible du visage, la métrique et une meilleure estimation de la taille et de la forme du modèle du visage voire également du modèle de la paire de lunettes.

Dès lors où le visage de l'individu 120, ou du moins seulement la paire de lunettes 110, est suivi par le procédé 200 décrit précédemment, un effacement de la paire de lunettes 110 porté par l'individu 120 dans le flux vidéo peut être effectué en se référant notamment à la technique décrite dans la demande de brevet internationale publiée sous le numéro WO 2018/002533. Un essayage virtuel d'une nouvelle paire de lunettes peut en outre être effectué.

Il convient de souligner que le procédé 200 de suivi étant plus efficace, l'effacement de la paire de lunettes dans l'image en occultant la paire de lunettes portée est effectué de manière plus réaliste car la position de la paire de lunettes est déterminée plus précisément par rapport à la caméra par le présent procédé de suivi.

Il est également envisageable grâce au procédé de suivi ici décrit de modifier tout ou partie de la paire de lunettes portée par l'individu, en effectuant par exemple un changement de couleur, de teinte des verres, un ajout d'un élément telle qu'une sérigraphie, etc.

Le procédé 200 de suivi peut ainsi être inclus dans un procédé de réalité augmentée.

Il convient de souligner que le procédé 200 de suivi peut également être utilisé dans un procédé de mesure d'un paramètre optique, tel que celui décrit dans la demande internationale de brevet publiée sous le numéro WO 2019/020521. En utilisant le procédé 200 de suivi, la mesure d'un paramètre optique peut être plus précise car les modèles paramétriques de la paire de lunettes et du visage sont résolus conjointement dans un même référentiel, ce qui n'est pas le cas dans les techniques antérieures où chaque modèle est optimisé indépendamment sans tenir compte des contraintes de positionnement relatif du modèle de la paire de lunettes et du modèle du visage.

### Détails du procédé mis en œuvre

L'algorithme présenté dans la présente section correspond à une mise en œuvre générique d'une partie d'un procédé de suivi objet de l'exemple détaillé précédemment. Cette partie correspond notamment à la résolution des paramètres, notamment de pose et de configuration/morphologie, du modèle du visage et du modèle de la paire de lunettes par rapport à des points détectés dans au moins un flux d'images (étape 220 ci-dessus) et à leur mise à jour (étape 235 ci-dessus). Il convient de souligner que ces deux étapes sont généralement basées sur une même équation résolue sous contrainte. Les modes morphologiques du modèle du visage et du modèle de la paire de lunettes peuvent également être résolus au cours de cette partie.

L'intérêt de résoudre en même temps le modèle du visage et le modèle de la paire de lunettes est d'apporter de nouvelles contraintes de collision ou de proximité entre le modèle du visage et le modèle de la paire de lunettes. En effet, il est ainsi assuré d'une part que les deux maillages, correspondant chacun à un modèle distinct, ne s'interpénètrent pas entre eux mais également qu'il y ait au moins des points qui sont en collision, ou à proximité, entre les deux maillages, notamment au niveau des oreilles et du nez de l'individu. Il convient de souligner qu'un des problèmes majeurs lors de la résolution de la pose d'un modèle du visage correspond aux positionnements des points au niveau des tempes dont l'emplacement est rarement déterminé précisément par le détecteur de points, usuellement mis en œuvre. L'utilisation des branches des lunettes qui sont souvent bien plus visibles dans l'image et physiquement contre les tempes est par conséquent avantageux.

Il convient de souligner qu'il est difficile de mettre en place un algorithme de collision à l'intérieur d'une minimisation car les deux modèles utilisés sont des modèles paramétriques, et par conséquent déformables. Les deux modèles se déformant à chaque itération les points de contact peuvent alors être distincts d'une itération à l'autre.

Dans le présent exemple non limitatif de l'invention, n caméras calibrées sont considérées, chacune acquérant *p* vues, à savoir *p* images. Il convient de souligner que les paramètres intrinsèques de chaque caméra et leur position relative sont connus. La position et l'orientation du visage est néanmoins à déterminer pour chacune des vues. Le modèle paramétrique 3D du visage utilisé, noté *M_{f}*, est un maillage composé de points 3D p3D déformables linéairement grâce à *v* paramètres notés *α*_{*k,k*=1..*v*}. Ainsi, chaque point 3D de ce maillage s'écrit sous la forme d'une combinaison linéaire :
[Math 1] $p 3 D _ {f}_{j} \left({α}_{1},…,{α}_{v}\right) = m 3 D _ {f}_{j} + {\sum }_{k = 1}^{v} {α}_{k} mode _ {f}_{j}^{k}$
où *m*3*Dⱼ* désigne le j^{ème} point moyen du modèle et ${mode}_{j}^{k}$ le j^{ème} vecteur du k^{ème} mode du modèle. L'indice *_f* est ajouté à *m*3*Dⱼ*, *p*3*D* et ${mode}_{j}^{k}$ pour indiquer que le modèle utilisé est celui du visage. Une équation similaire pour le modèle de la paire de lunettes noté *M_{g}* peut s'écrire :
[Math 2] $p 3 D _ {g}_{j} \left({β}_{1},{β}_{2},…,{β}_{μ}\right) = m 3 D _ {g}_{j} + {\sum }_{k = 1}^{μ} {β}_{k} mode _ {g}_{j}^{k}$
où *β*_{*k,k*=1...*µ*}.correspondent à *µ* paramètres du modèle paramétrique de la paire de lunettes *M_{g}*

Le visage en 3D est replacé dans un premier temps dans un repère tridimensionnel, dit repère monde, pour chacune des *p* acquisitions. Le repère monde peut par exemple correspondre au repère de la caméra ou à un repère de l'un des deux modèles. Les positions et orientations du modèle du visage sont initialement inconnues et par conséquent recherchées lors de la minimisation, ce qui correspond à une phase de régression des points du modèle du visage avec des points caractéristiques détectés à l'image.

Avant d'effectuer cette régression, le modèle *M_{g}* de la paire de lunettes est positionné sur le modèle *M_{f}* du visage. À cet effet, les points p3D_g du modèle de la paire de lunettes peuvent s'écrire dans le repère du visage en tenant compte d'une matrice *R*_*g* de rotation 3D et d'un vecteur T_g de translation. [Math 3] $p 3 D _ g _ {p}_{j} = \left[\begin{matrix}R _ g & T _ g \\ 0 0 0 & 1\end{matrix}\right] p 3 D _ {g}_{j} \left({β}_{1},{β}_{2},…,{β}_{μ}\right)$

La régression aboutit ensuite à une pose en orientation et en translation du modèle de visage dans le repère dans le repère de la vue *l* d'une des caméras, correspondant ici au repère monde. [Math 4] $p 3 D _ {f}_{j}^{l} = \left[\begin{matrix}R _ {f}_{l} & T _ {f}_{l} \\ 0 0 0 & 1\end{matrix}\right] p 3 D _ {f}_{j} \left({α}_{1},…,{α}_{v}\right)$
où R représente une matrice de rotation 3D, T un vecteur de translation et *l* une vue d'une caméra

Une fonction de projection d'un modèle p3D dans l'image i utilisée au cours du procédé est notée :
[Math 5] ${Proj}^{i} \left(p3D\right) ∼ {K}^{i} \left[{R}^{i}{T}^{i}\right] p 3 D$
où *Kⁱ* correspond à la matrice de calibrage de l'image *i. Rⁱ* et *Tⁱ* correspondent respectivement à une matrice de rotation et à un vecteur de translation entre le repère monde et le repère de la caméra ayant acquis l'image i. Le symbole ~ désigne quant à lui une égalité à un facteur d'échelle près. Cette égalité peut notamment se traduire par le fait que la dernière composante de la projection est égale à 1.

Lors de la résolution de la pose des modèles de la représentation du visage, il existe cinq types de contraintes :
- les contraintes 2D visage ;
- les contraintes 2D lunettes ;
- les contraintes 3D visage - lunettes ;
- les contraintes 3D visage, correspondant par exemple à une distance interpupillaire PD, à un écart entre les tempes, à une taille moyenne d'iris ou à un mélange de distributions de plusieurs contraintes de taille. Un mélange de distribution peut correspondre à un mélange de deux distributions gaussiennes autour de la taille d'un iris et de la distance interpupillaire. La combinaison de ces contraintes peut faire appel à une formulation de type filtre GH ;
- les contraintes 3D des lunettes, correspondant par exemple à une dimension connue issue du marquage sur la monture, couramment appelée par le terme anglais « frame marking ».

Les contraintes 2D du visage sont basées sur un appariement des points du modèle 3D à des points 2D dans l'image du visage pour au moins une vue et pour au moins une caméra. Préférentiellement, cet appariement est effectué pour chaque vue et pour chaque caméra. Il convient de souligner que les appariements peuvent être fixes pour les points du visage non compris sur le contour du visage dans l'image ou glissant le long de lignes de niveaux pour les points du contour du visage. Ce degré de liberté dans l'appariement d'un point du contour du visage avec un point de l'image permet notamment d'améliorer la stabilité de la pose du modèle 3D du visage par rapport à l'image, offrant ainsi une meilleure continuité de pose du modèle 3D du visage entre deux images successives.

L'appariement d'un point du modèle 3D du visage avec un point 2D de l'image peut se traduire mathématiquement par l'équation suivante :
[Math 6] $p 3 {D}_{{f}_{{φ}_{j , i , l}}} ↔ p 2 {D}_{{f}_{{σ}_{j , i , l}}}$
où *φ_{j,i,l}* et *σ_{j,i,l}* représentent respectivement un indice d'un point 3D du modèle paramétrique *Mf* du visage et un indice d'un point 2D du visage dans les images pour une vue i et une caméra *l*.

Les contraintes 2D des lunettes sont basées sur un appariement des points 3D du modèle de la paire de lunettes avec des points 2D des lunettes dans une image en utilisant notamment les contours des masques dans les images. [Math 7] $p 3 D _ {g}_{{θ}_{j , i , l}} ↔ p 2 D _ {g}_{{ω}_{j , i , l}}$
où *θ_{j,i,l}* et *ω_{j,i,l}* représentent respectivement un indice d'un point 3D du modèle paramétrique *Mg* de la paire de lunettes et un indice d'un point 2D de la paire de lunettes dans les images pour une vue i et une caméra *l*.

Les contraintes 3D visage - lunettes sont basées sur un appariement des points 3D du modèle du visage et des points 3D du modèle de la paire de lunettes, dont la distance est définie par une contrainte de proximité, voire de collision (distance nulle). Une fonction d'influence peut être appliquée pour calculer la distance de collision avec par exemple un poids plus important pour les distances négatives par rapport à la normale de la surface du modèle du visage orientée vers l'extérieur du modèle du visage. Il convient de souligner que pour certains points, la contrainte peut être uniquement sur une partie des coordonnées, comme par exemple selon un axe pour la relation entre les tempes du visage et les branches de la paire de lunettes.

L'appariement des points 3D du modèle du visage et des points 3D du modèle de la paire de lunettes peut se traduire mathématiquement par l'équation suivante :
[Math 8] $p 3 D _ {f}_{{ρ}_{j}} ↔ p 3 D _ {g}_{{τ}_{j}}$
où *ρⱼ* et*τⱼ* représentent respectivement un indice d'un point 3D du modèle paramétrique *Mf* du visage et un indice d'un point 3D du modèle paramétrique *Mg* de la paire de lunettes.

Les contraintes 3D sur le visage sont basées sur une distance connue du visage, préalablement mesurée, comme par exemple la distance interpupillaire (distance entre le centre de chaque pupille, correspondant également à la distance entre le centre de rotation de chaque œil). Une distance métrique peut ainsi être appariée à un couple de points. [Math 9] $\left(p3D_{f}_{{t}_{j}},p3D_{f}_{{u}_{j}}\right) ↔ {dist}_{{t}_{j} {u}_{j}}$
où *tⱼ* et *uⱼ* représentent chacun un indice d'un point 3D distinct du modèle paramétrique *Mf* du visage.

Les contraintes 3D sur la paire de lunettes sont basées sur une distance connue du modèle de la paire de lunettes portée par l'individu, telle que la taille d'un verre (par exemple selon la norme BOXING ou la norme DATUM), la taille du pont ou la taille des branches. Cette distance peut notamment être traduite du marquage de la monture, généralement située à l'intérieur d'une branche, couramment appelée *« frame marking ».* Une distance métrique peut alors être appariée à un couple de points du modèle de la paire de lunettes. [Math 10] $\left(p3D_{g}_{{v}_{j}},p3D_{g}_{{w}_{j}}\right) ↔ {dist}_{{v}_{j} {w}_{j}}$
où *vⱼ* et *wⱼ* représentent chacun un indice d'un point 3D distinct du modèle paramétrique *Mg* de la paire de lunettes.

Les données d'entrée de l'algorithme sont ainsi :
- *p* images issues de n caméras d'une personne portant une paire de lunettes ;
- points 2D caractéristiques du visage, détectés dans une image ;
- appariements 2D ou 3D pour une partie des points, éventuellement évalués à chaque itération dans le cas des points dits glissants (ex : le long des lignes de niveaux) ;
- le masque de la paire de lunettes dans au moins une image ;
- la matrice de calibrage et la pose de chaque caméra.

L'algorithme va permettre de calculer les données de sortie suivantes :
- les *p* poses de l'avatar : *R_{fl}*, *T_{fl} ;*
- les *v* modes du modèle paramétrique du visage : *α*₁, *α*₂, ..., *αᵥ ;*
- la pose du modèle de la paire de lunettes par rapport au modèle du visage : *R_{g}, T_{g}* ;
- les *µ* modes du modèle paramétrique de la paire de lunettes : *β*₁, *β*₂, ..., *β_{µ}*.

A cet effet, l'algorithme procède selon les étapes suivantes :
- effectuer les appariements des points *φ_{j,i,l}* ↔ *σ_{j,i,l}* pour les contraintes 2D du visage ;
- effectuer les appariements des points *θ_{j,i,l}* ↔ *ω_{j,i,l}* pour les contraintes 2D de la paire de lunettes ;
- effectuer les appariements des points *ρⱼ* ↔ *τⱼ* pour les contraintes 3D entre le modèle du visage et le modèle de la paire de lunettes ;
- effectuer les appariements des points *tⱼ* ↔ *uⱼ* et les associer à une distance métrique *distₜⱼᵤⱼ* pour établir les contraintes 3D sur le modèle du visage;
- effectuer les appariements des points *vⱼ* ↔ *wⱼ* et les associer à une distance métrique *dist_{vjwj}* pour établir les contraintes 3D sur le modèle de la paire de lunettes ;
- résoudre l'équation mathématique suivante.
[Math 11] $\begin{matrix}{min}_{{α}_{1} , … , {α}_{ν} , {R}_{{f}_{i}} , {T}_{{j}_{i}} , {β}_{t} , … , {β}_{μ} , R _ g , T _ g} \frac{{γ}_{1}}{# \left(visi==1\right)} \left({\sum }_{i = 1}^{n} {\sum }_{i = 1}^{p} {\sum }_{j = 1}^{{m}_{1}} visi \left(p2D_{f}_{{σ}_{j , i , i}}\right){\left‖\begin{matrix}{Proj}^{i} \left(\left[\begin{matrix}{R}_{{f}_{l}} & {T}_{{f}_{l}} \\ 0 0 0 & 1\end{matrix}\right]p3D_{f}_{{φ}_{j , i , i}}\left({α}_{1},…,{α}_{v}\right)\right) \\ - p 2 D _ {f}_{{σ}_{j , i , i}}\end{matrix}\right‖}_{2}\right) \\ + \frac{{γ}_{2}}{# \left(visi==1\right)} \left({\sum }_{i = 1}^{n} {\sum }_{i = 1}^{p} {\sum }_{j = 1}^{{m}_{2}} visi \left(p2D_{g}_{{ω}_{j , i , l}}\right) {\left‖\begin{matrix}{Proj}^{i} \left(\left[\begin{matrix}{R}_{{f}_{↓}} & {T}_{{f}_{l}} \\ 0 0 0 & 1\end{matrix}\right]\left[\begin{matrix}R _ g & T _ g \\ 0 0 0 & 1\end{matrix}\right]p3D_{g}_{{θ}_{j , i , l}}\left({β}_{1},{β}_{2},…,{β}_{μ}\right)\right) \\ - p 2 D _ {g}_{{ω}_{j , i , l}}\end{matrix}\right‖}_{2}\right) + {γ}_{3} \left({\sum }_{j = 1}^{{m}_{3}} {\left‖p3D_{f}_{{ρ}_{j}}\left({α}_{1},…,{α}_{v}\right)-\left[\begin{matrix}R _ g & T _ g \\ 0 0 0 & 1\end{matrix}\right]p3D_{g}_{{τ}_{j}}\left({β}_{1},{β}_{2},…,{β}_{μ}\right)\right‖}_{2}\right) \\ + {γ}_{4} \left({\sum }_{j = 1}^{{m}_{4}} {\left‖{\left‖p3D_{f}_{{t}_{j}}\left({α}_{1},…,{α}_{v}\right)-p3D_{f}_{{u}_{j}}\left({α}_{1},…,{α}_{v}\right)\right‖}_{2}-{dist}_{{t}_{j} {u}_{j}}\right‖}_{2}\right) \\ + {γ}_{5} \left({\sum }_{j = 1}^{{m}_{5}} {\left‖{\left‖p3D_{g}_{{v}_{j}}\left({β}_{1},{β}_{2},…,{β}_{μ}\right)-p3D_{g}_{{w}_{j}}\left({β}_{1},{β}_{2},…,{β}_{μ}\right)\right‖}_{2}-{dist}_{{v}_{j} {w}_{j}}\right‖}_{2}\right)\end{matrix}$
où *γ*₁*,γ*₂*,γ*₃*,γ*₄*,γ*₅ sont des poids entre chaque bloc de contrainte, *visi* est une fonction indiquant si un point p2D est visible dans l'image, c'est-à-dire non occulté par le modèle de visage *Mf* ou par le modèle de la paire de lunettes *Mg,* #(*visi ==* 1) correspond au nombre de points visibles.

Dans des variantes de ce mode de mise en œuvre particulier de l'invention, la focale de la caméra fait partie des paramètres à optimiser. En effet, dans les cas où l'acquisition des images est effectuée par une caméra inconnue, certaines images acquises sont préalablement recadrées ou redimensionnées. Auquel cas, il est préférable de laisser la focale de la caméra en tant que degré de liberté lors de la minimisation.

Dans des variantes de ce mode de mise en œuvre particulier de l'invention, les matrices de variance et de covariance qui représentent les axes et valeurs d'incertitudes/confiance des paramètres pour les équations de contraintes de collisions entre le modèle du visage et le modèle de la paire de lunettes, sont prises en compte lors de la résolution.

Dans des variantes de ce mode de mise en œuvre particulier de l'invention, certains paramètres de la pose du modèle de la paire de lunettes par rapport au modèle du visage sont figés. Cela peut être la traduction d'une hypothèse d'alignement entre le modèle de la paire de lunettes et le modèle du visage. Dans ce cas, seule la rotation selon l'axe des x, soit selon un axe perpendiculaire au plan sagittal, ainsi que la translation en y et z, soit dans le plan sagittal, sont calculées. La fonction de coût, représentée par [Math 11] peut être simplifiée, ce qui permet d'obtenir une convergence plus facile vers le résultat. De cette manière, il est également possible d'obtenir des résultats très satisfaisants pour des visages fortement asymétriques où la paire de lunettes peut être positionnée différemment par rapport à un visage symétrique, par exemple légèrement inclinée d'un côté du visage.

### Exemple d'un modèle paramétrique d'une paire de lunettes

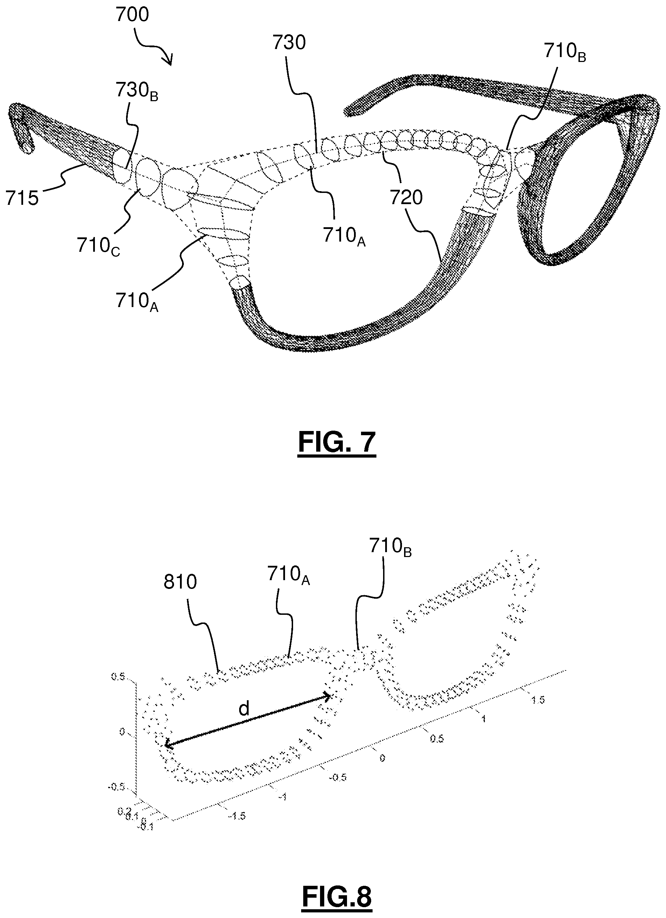

Chaque paire de lunettes comporte des éléments communs tels que les verres, le pont et les branches. Un modèle paramétrique (3DMM) 700 de paire de lunettes, tel que représenté en figure 7, peut ainsi être défini comme un ensemble de sections 710 reliées entre elles par des faces triangulaires 715 définies en amont.

Les faces triangulaires 715 forment une enveloppe convexe 720 dont une partie n'est pas représentée sur la figure 7.

Chacune des sections 710, définie par un même nombre de points, se situe avantageusement au même endroit sur l'ensemble des modèles de paire de lunettes.

En outre, chaque section 710 coupe la paire selon un plan perpendiculaire au squelette 730.

Trois types de sections peuvent ainsi être définies :
- les sections 710_{A} autour des verres, paramétrisées par exemple par un angle par rapport à un plan de référence perpendiculaire au squelette d'un cercle, afin d'avoir une section toute les n degrés ;
- les sections 710_{B} du pont, parallèles au plan de référence
- les sections 710c des branches, le long du squelette 730_{B} des branches

Il convient de souligner que dans le cas d'une paire sans cercle autour d'un verre, couramment qualifiée par le terme anglais « *rimless* », ou dans le cas d'une paire dite demi-cerclées ou « semi-rimless », c'est-à-dire qu'un cercle entoure qu'une partie d'un verre, tout ou partie des sections 710_{A} autour des verres présente qu'un seul point correspondant à la réunion de l'ensemble des points d'une même section 710_{A}.

Par ailleurs, l'analyse en composantes principales (ACP) utilisée lors de l'alignement du modèle 700 de la paire de lunettes avec la représentation de la paire de lunettes dans l'image, impose un nombre de points communs. A cet effet, des points qui se trouvent sur l'enveloppe 720 convexe du modèle de la paire de lunettes sont choisis afin d'assurer de retrouver dans l'image l'ensemble des pixels appartenant à la paire de lunettes alignée.

Pour permettre de retrouver des lumières dans la paire de lunettes, comme par exemple dans le cas d'une paire de lunettes présentant un double pont, un template de modèle de paire de lunettes, par exemple avec un double pont, peut être choisi au préalable pour s'adapter au près de la paire de lunettes.

Dans la mesure où un point du modèle paramétrique, référencé avec un indice donné, se trouve constamment au même endroit relatif sur le modèle de la paire de lunettes, une définition de la distance connue entre deux points peut être facilitée. Cette distance connue peut être obtenue par le « *frame marking »* inscrit sur une paire de lunettes, qui permet de définir la largeur des verres, la largeur du pont ou encore la longueur des branches.

Ces informations peuvent alors être imposées dans la résolution du modèle 700 de lunettes en sélectionnant les points correspondants, comme illustré par la figure 8. Dans la figure 8, seuls les points 810 caractérisant les contours des sections 710 de la face de la paire de lunettes sont représentés, et d correspond à la largeur d'un verre tel que défini grâce notamment au « *frame marking ».*

Dans une variante de l'alignement visage et lunettes, un grand nombre de visages et un grand nombre de lunettes sont générés à partir des deux modèles paramétriques respectifs du visage et de la paire de lunettes. L'algorithme de positionnement automatique est ensuite utilisé pour positionner chaque modèle de paire de lunettes sur chaque modèle de visage. Avantageusement une génération de bruit et des statistiques de positionnement différentes - lunettes au bout du nez, enfoncement des plaquettes, positionnement lâche sur les tempes, etc. - sont utilisés pour positionner automatiquement les paires de lunettes sur les visages. Un nouveau modèle paramétrique pour la paire de lunettes et pour le visage est ensuite calculé à partir de l'ensemble des points des modèles du visage et de la paire de lunettes. Ce nouveau modèle paramétrique garantit la collision et le parfait positionnement de la paire de lunettes sur le visage, ce qui simplifie la résolution. En effet, une seule transformation est recherchée, ce qui correspond au calcul de six paramètres au lieu de douze, et les équations de collision sont retirées. Cependant, un plus grand nombre de modes sont généralement estimés dans ce cas car ce sont eux qui encodent ces contraintes.

## Revendications

1. Procédé (200) de suivi d'un visage (125) d'un individu (120) dans un flux vidéo acquis par un dispositif d'acquisition d'images (130), le visage portant une paire de lunettes (110), le flux vidéo comprenant une pluralité d'images acquises successivement, le procédé de suivi comprenant une étape (220, 235) d'évaluation de paramètres d'une représentation du visage comprenant un modèle de la paire de lunettes et un modèle du visage de telle sorte que ladite représentation du visage se superpose à l'image du visage dans le flux vidéo, **caractérisé en ce que** tout ou partie des paramètres de la représentation sont évalués en tenant compte d'au moins une contrainte de proximité entre au moins un point du modèle du visage et au moins un point du modèle de la paire de lunettes.

2. Procédé de suivi selon la revendication précédente, dans lequel les paramètres de la représentation comprennent des valeurs externes à la représentation du visage et des valeurs internes à la représentation du visage, les valeurs externes comprenant une position tridimensionnelle et une orientation tridimensionnelle de la représentation du visage par rapport au dispositif d'acquisition d'images, les valeurs internes comprenant une position tridimensionnelle et une orientation tridimensionnelle du modèle de la paire de lunettes par rapport au modèle du visage, lesdits paramètres étant évalués par rapport à une pluralité de points caractéristiques de ladite représentation du visage, préalablement détectés dans une image du flux vidéo, dite première image, ou dans un jeu d'images acquises simultanément par une pluralité de dispositif d'acquisition d'images, le jeu d'images comprenant ladite première image.

3. Procédé de suivi selon l'une quelconque des revendications précédentes, dans lequel tout ou partie des paramètres de la représentation sont mis à jour par rapport à la position de tout ou partie des points caractéristiques, suivis ou détectés, dans une deuxième image du flux vidéo ou dans une deuxième série d'images acquises simultanément par la pluralité de dispositif d'acquisition d'images, le deuxième jeu d'images comprenant ladite deuxième image.

4. Procédé de suivi selon l'une quelconque des revendications précédentes, dans lequel tout ou partie des paramètres de la représentation sont également évalués en tenant compte d'au moins une contrainte de proximité entre un point tridimensionnel d'un des modèles inclus dans la représentation du visage et au moins un point, ou une ligne de niveau, inclus(e) dans au moins une image du flux vidéo.

5. Procédé de suivi selon l'une quelconque des revendications précédentes, dans lequel tout ou partie des paramètres de la représentation sont également évalués en tenant compte d'au moins une contrainte de dimension d'un des modèles inclus dans la représentation du visage.

6. Procédé de suivi selon l'une quelconque des revendications précédentes, dans lequel le procédé comprend une étape d'appariement de deux points distincts appartenant soit à l'un des deux modèles compris dans la représentation du visage, soit chacun à un modèle distinct parmi les modèles compris dans la représentation du visage.

7. Procédé de suivi selon l'une quelconque des revendications précédentes, dans lequel le procédé comprend une étape préalable d'appariement d'un point d'un des deux modèles compris dans la représentation du visage avec au moins un point d'une image acquise par un dispositif d'acquisition d'images.

8. Procédé de suivi selon l'une quelconque des revendications précédentes, dans lequel, au cours de l'évaluation des paramètres de la représentation, un alignement du modèle de la paire de lunettes avec une image de la paire de lunettes dans le flux vidéo est effectué consécutivement à un alignement du modèle du visage avec une image du visage dans le flux vidéo.

9. Procédé de suivi selon la revendication précédente, dans lequel l'alignement du modèle du visage est effectué en minimisant la distance entre des points caractéristiques du visage détectés dans l'image du visage et des points caractéristiques du modèle du visage projetés dans ladite image du visage.

10. Procédé de suivi selon l'une quelconque des revendications 8 à 9, dans lequel l'alignement du modèle de la paire de lunettes est effectué en minimisant la distance entre au moins une partie du contour de la paire de lunettes dans l'image et une partie de contour similaire du modèle de la paire de lunettes projeté dans ladite image.

11. Procédé de suivi selon l'une quelconque des revendications précédentes, dans lequel les paramètres de la représentation comprennent tout ou partie de la liste suivante :
• une position tridimensionnelle de la représentation du visage ;
• une orientation tridimensionnelle de la représentation du visage ;
• une taille du modèle de la paire de lunettes ;
• une taille du modèle du visage ;
• une position tridimensionnelle relative entre le modèle de la paire de lunettes et le modèle du visage ;
• une orientation tridimensionnelle relative entre le modèle de la paire de lunettes et le modèle du visage ;
• un ou des paramètre(s) de configuration du modèle de la paire de lunettes ;
• un ou des paramètre(s) de configuration du modèle du visage ;
• un ou des paramètre(s) de la caméra.

12. Procédé de suivi selon la revendication précédente, comprenant des étapes de :
• détection d'une pluralité de points du visage dans une première image du flux vidéo ;
• initialisation du jeu de paramètres du modèle du visage par rapport à l'image du visage dans ladite première image initiale ;
• détection d'une pluralité de points d'une paire de lunettes portée par le visage de l'individu dans une deuxième image du flux vidéo, dite deuxième image initiale, la deuxième image initiale étant soit postérieure ou antérieure à la première image initiale dans le flux vidéo, soit identique à la première image dans le flux vidéo ;
• initialisation du jeu de paramètres du modèle de la paire de lunettes par rapport à l'image de la paire de lunettes dans ladite deuxième image initiale.

13. Procédé de suivi selon la revendication précédente, dans lequel l'initialisation des paramètres du modèle du visage est effectuée par l'intermédiaire d'une méthode d'apprentissage profond analysant tout ou partie des points détectés du visage.

14. Procédé de suivi selon la revendication précédente, dans lequel la méthode d'apprentissage profond détermine également une position initiale du modèle du visage dans le repère tridimensionnel.

15. Procédé de suivi selon l'une quelconque des revendications précédentes, comprenant également une étape de détermination d'une échelle de l'image de la paire de lunettes portée par le visage de l'individu par l'intermédiaire d'une dimension dans l'image d'un élément de taille connue de la paire de lunettes.

16. Procédé de suivi selon la revendication précédente, dans lequel la détermination de l'échelle est effectuée par l'intermédiaire d'une reconnaissance préalable de la paire de lunettes portée par le visage de l'individu.

17. Procédé de suivi selon l'une quelconque des revendications précédentes, dans lequel des images acquises par un deuxième dispositif d'acquisition d'images sont utilisées pour évaluer les paramètres de la représentation.

18. Procédé de suivi selon l'une quelconque des revendications précédentes, dans lequel le modèle de la paire de lunettes de la représentation correspond à une modélisation préalable de ladite paire de lunettes, et varie uniquement en déformation.

19. Procédé de réalité augmentée comprenant des étapes de :
• acquisition d'au moins un flux d'images d'un individu portant une paire de lunettes sur son visage par au moins un dispositif d'acquisition d'images ;
• suivi du visage de l'individu par un procédé de suivi selon l'une quelconque des revendications 1 à 18, une position et une orientation d'une représentation du visage ;
• modification de tout ou partie des images dudit ou d'un desdits flux d'images, appelé flux vidéo principal, acquis par le dispositif d'acquisition d'images ou par l'un des dispositifs d'acquisition d'images, appelé dispositif principal d'acquisition d'images, grâce à la représentation du visage se superposant en temps réel au visage de l'individu sur le flux vidéo principal ;
• affichage sur un écran du flux vidéo principal modifié précédemment.

20. Dispositif électronique comportant une mémoire informatique stockant des instructions d'un procédé selon l'une quelques des revendications précédentes.

## Patentansprüche

1. Verfahren (200) zum Verfolgen eines Gesichts (125) eines Individuums (120) in einem Videostrom, der von einer Bilderfassungsvorrichtung (130) erfasst wird, wobei das Gesicht eine Brille (110) trägt, wobei der Videostrom eine Vielzahl von nacheinander erfassten Bildern umfasst, wobei das Verfolgungsverfahren einen Schritt (220, 235) des Ermittelns von Parametern einer Darstellung des Gesichts umfasst, die ein Modell der Brille und ein Modell des Gesichts umfasst, derart, dass sich die genannte Darstellung des Gesichts dem Bild des Gesichts im Videostrom überlagert, **dadurch gekennzeichnet, dass** alle oder ein Teil der Parameter der Darstellung unter Berücksichtigung mindestens einer Nähebedingung zwischen mindestens einem Punkt des Modells des Gesichts und mindestens einem Punkt des Modells der Brille ermittelt werden.

2. Verfolgungsverfahren nach dem vorhergehenden Anspruch, wobei die Parameter der Darstellung Werte, die extern zur Darstellung des Gesichts sind, und Werte, die intern zur Darstellung des Gesichts sind, umfassen, wobei die externen Werte eine dreidimensionale Position und eine dreidimensionale Orientierung der Darstellung des Gesichts in Bezug auf die Bilderfassungsvorrichtung umfassen, wobei die internen Werte eine dreidimensionale Position und eine dreidimensionale Orientierung des Modells der Brille in Bezug auf das Modell des Gesichts umfassen, wobei die genannten Parameter in Bezug auf eine Vielzahl charakteristischer Punkte der genannten Darstellung des Gesichts ermittelt werden, die zuvor in einem Bild des Videostroms, das als erstes Bild bezeichnet wird, oder in einem Satz von Bildern, die gleichzeitig von einer Vielzahl von Bilderfassungsvorrichtungen erfasst werden, detektiert wurden, wobei der Satz von Bildern das genannte erste Bild umfasst.

3. Verfolgungsverfahren nach einem der vorhergehenden Ansprüche, wobei alle oder ein Teil der Parameter der Darstellung in Bezug auf die Position aller oder eines Teils der charakteristischen Punkte, die in einem zweiten Bild des Videostroms oder in einer zweiten Reihe von gleichzeitig von der Vielzahl von Bilderfassungsvorrichtungen erfassten Bildern verfolgt oder detektiert werden, aktualisiert werden, wobei der zweite Satz von Bildern das genannte zweite Bild umfasst.

4. Verfolgungsverfahren nach einem der vorhergehenden Ansprüche, wobei alle oder ein Teil der Parameter der Darstellung ebenfalls unter Berücksichtigung mindestens einer Nähebedingung zwischen einem dreidimensionalen Punkt eines der in der Darstellung des Gesichts enthaltenen Modelle und mindestens einem Punkt oder einer Niveaulinie, der bzw. die in mindestens einem Bild des Videostroms enthalten ist, ermittelt werden.

5. Verfolgungsverfahren nach einem der vorhergehenden Ansprüche, wobei alle oder ein Teil der Parameter der Darstellung ebenfalls unter Berücksichtigung mindestens einer Abmessungsbedingung eines der in der Darstellung des Gesichts enthaltenen Modelle ermittelt werden.

6. Verfolgungsverfahren nach einem der vorhergehenden Ansprüche, wobei das Verfahren einen Schritt der Zuordnung zweier verschiedener Punkte umfasst, die entweder zu einem der beiden in der Darstellung des Gesichts enthaltenen Modelle gehören oder jeweils zu einem verschiedenen Modell unter den in der Darstellung des Gesichts enthaltenen Modellen gehören.

7. Verfolgungsverfahren nach einem der vorhergehenden Ansprüche, wobei das Verfahren einen vorherigen Schritt der Zuordnung eines Punktes eines der beiden in der Darstellung des Gesichts enthaltenen Modelle zu mindestens einem Punkt eines von einer Bilderfassungsvorrichtung erfassten Bildes umfasst.

8. Verfolgungsverfahren nach einem der vorhergehenden Ansprüche, wobei im Verlauf der Ermittlung der Parameter der Darstellung eine Ausrichtung des Modells der Brille an einem Bild der Brille im Videostrom im Anschluss an eine Ausrichtung des Modells des Gesichts an einem Bild des Gesichts im Videostrom durchgeführt wird.

9. Verfolgungsverfahren nach dem vorhergehenden Anspruch, wobei die Ausrichtung des Modells des Gesichts durch Minimieren des Abstands zwischen charakteristischen Punkten des Gesichts, die im Bild des Gesichts detektiert werden, und charakteristischen Punkten des Modells des Gesichts, die in das genannte Bild des Gesichts projiziert werden, durchgeführt wird.

10. Verfolgungsverfahren nach einem der Ansprüche 8 bis 9, wobei die Ausrichtung des Modells der Brille durch Minimieren des Abstands zwischen mindestens einem Teil der Kontur der Brille im Bild und einem ähnlichen Konturteil des in das genannte Bild projizierten Modells der Brille durchgeführt wird.

11. Verfolgungsverfahren nach einem der vorhergehenden Ansprüche, wobei die Parameter der Darstellung alle oder einen Teil der folgenden Liste umfassen:
• eine dreidimensionale Position der Darstellung des Gesichts;
• eine dreidimensionale Orientierung der Darstellung des Gesichts;
• eine Größe des Modells der Brille;
• eine Größe des Modells des Gesichts;
• eine relative dreidimensionale Position zwischen dem Modell der Brille und dem Modell des Gesichts;
• eine relative dreidimensionale Orientierung zwischen dem Modell der Brille und dem Modell des Gesichts;
• einen oder mehrere Konfigurationsparameter des Modells der Brille;
• einen oder mehrere Konfigurationsparameter des Modells des Gesichts;
• einen oder mehrere Parameter der Kamera.

12. Verfolgungsverfahren nach dem vorhergehenden Anspruch, umfassend Schritte:
• Detektion einer Vielzahl von Punkten des Gesichts in einem ersten Bild des Videostroms;
• Initialisierung des Satzes von Parametern des Modells des Gesichts in Bezug auf das Bild des Gesichts in dem genannten ersten Anfangsbild;
• Detektion einer Vielzahl von Punkten einer vom Gesicht des Individuums getragenen Brille in einem zweiten Bild des Videostroms, das als zweites Anfangsbild bezeichnet wird, wobei das zweite Anfangsbild entweder nach oder vor dem ersten Anfangsbild im Videostrom liegt oder mit dem ersten Bild im Videostrom identisch ist;
• Initialisierung des Satzes von Parametern des Modells der Brille in Bezug auf das Bild der Brille in dem genannten zweiten Anfangsbild.

13. Verfolgungsverfahren nach dem vorhergehenden Anspruch, wobei die Initialisierung der Parameter des Modells des Gesichts mittels eines Verfahrens des tiefen Lernens durchgeführt wird, das alle oder einen Teil der detektierten Punkte des Gesichts analysiert.

14. Verfolgungsverfahren nach dem vorhergehenden Anspruch, wobei das Verfahren des tiefen Lernens ebenfalls eine Anfangsposition des Modells des Gesichts im dreidimensionalen Bezugssystem bestimmt.

15. Verfolgungsverfahren nach einem der vorhergehenden Ansprüche, ebenfalls umfassend einen Schritt der Bestimmung eines Maßstabs des Bildes der vom Gesicht des Individuums getragenen Brille mittels einer Abmessung, im Bild, eines Elements bekannter Größe der Brille.

16. Verfolgungsverfahren nach dem vorhergehenden Anspruch, wobei die Bestimmung des Maßstabs mittels einer vorherigen Erkennung der vom Gesicht des Individuums getragenen Brille durchgeführt wird.

17. Verfolgungsverfahren nach einem der vorhergehenden Ansprüche, wobei von einer zweiten Bilderfassungsvorrichtung erfasste Bilder verwendet werden, um die Parameter der Darstellung zu ermitteln.

18. Verfolgungsverfahren nach einem der vorhergehenden Ansprüche, wobei das Modell der Brille der Darstellung einer vorherigen Modellierung der genannten Brille entspricht und nur in der Verformung variiert.

19. Verfahren der erweiterten Realität, umfassend Schritte:
• Erfassen mindestens eines Bildstroms eines eine Brille auf seinem Gesicht tragenden Individuums durch mindestens eine Bilderfassungsvorrichtung;
• Verfolgen des Gesichts des Individuums durch ein Verfolgungsverfahren nach einem der Ansprüche 1 bis 18, eine Position und eine Orientierung einer Darstellung des Gesichts;
• Modifizieren aller oder eines Teils der Bilder des genannten Bildstroms oder eines der genannten Bildströme, der als Haupt-Videostrom bezeichnet wird und von der Bilderfassungsvorrichtung oder von einer der Bilderfassungsvorrichtungen, die als Haupt-Bilderfassungsvorrichtung bezeichnet wird, erfasst wird, mittels der Darstellung des Gesichts, die sich in Echtzeit dem Gesicht des Individuums auf dem Haupt-Videostrom überlagert;
• Anzeigen des zuvor modifizierten Haupt-Videostroms auf einem Bildschirm.

20. Elektronische Vorrichtung, umfassend einen Computerspeicher, der Anweisungen eines Verfahrens nach einem der vorhergehenden Ansprüche speichert.

## Claims

1. Method (200) for tracking a face (125) of an individual (120) in a video stream acquired by an image-acquisition device (130), the face wearing a pair of spectacles (110), the video stream comprising a plurality of images acquired successively, the tracking method comprising a step (220, 235) of evaluating parameters of a representation of the face comprising a model of the pair of spectacles and a model of the face so that said representation of the face is superimposed on the image of the face in the video stream, **characterized in that** all or some of the parameters of the representation are evaluated by taking account of at least one proximity constraint between at least one point of the model of the face and at least one point of the model of the pair of spectacles.

2. Tracking method according to the preceding claim, wherein the parameters of the representation comprise values external to the representation of the face and values internal to the representation of the face, the external values comprising a three-dimensional position and a three-dimensional orientation of the representation of the face with respect to the image-acquisition device, the internal values comprising a three-dimensional position and a three-dimensional orientation of the model of the pair of spectacles with respect to the model of the face, said parameters being evaluated with respect to a plurality of characteristic points of said representation of the face, previously detected in an image of the video stream, referred to as first image, or in a set of images acquired simultaneously by a plurality of image-acquisition devices, the set of images comprising said first image.

3. Tracking method according to any one of the preceding claims, wherein all or some of the parameters of the representation are updated with respect to the position of all or some of the characteristic points, tracked or detected, in a second image of the video stream or in a second series of images acquired simultaneously by the plurality of image-acquisition devices, the second set of images comprising said second image.

4. Tracking method according to any one of the preceding claims, wherein all or some of the parameters of the representation are also evaluated by taking account of at least one proximity constraint between a three-dimensional point of one of the models included in the representation of the face and at least one point, or a level line, included in at least one image of the video stream.

5. Tracking method according to any one of the preceding claims, wherein all or some of the parameters of the representation are also evaluated by taking account of at least one dimension constraint of one of the models included in the representation of the face.

6. Tracking method according to any one of the preceding claims, wherein the method comprises a step of pairing two distinct points belonging either to one of the two models included in the representation of the face, or each to a distinct model from the models included in the representation of the face.

7. Tracking method according to any one of the preceding claims, wherein the method comprises a prior step of pairing a point of one of the two models included in the representation of the face with at least one point of an image acquired by an image-acquisition device.

8. Tracking method according to any one of the preceding claims, wherein, during the evaluation of the parameters of the representation, an alignment of the model of the pair of spectacles with an image of the pair of spectacles in the video stream is implemented consecutively with an alignment of the model of the face with an image of the face in the video stream.

9. Tracking method according to the preceding claim, wherein the alignment of the model of the face is implemented by minimizing the distance between characteristic points of the face detected in the image of the face and characteristic points of the model of the face projected in said image of the face.

10. Tracking method according to any one of claims 8 to 9, wherein the alignment of the model of the pair of spectacles is implemented by minimizing the distance between at least a part of the contour of the pair of spectacles in the image and a similar contour part of the model of the pair of spectacles projected in said image.

11. Tracking method according to any one of the preceding claims, wherein the parameters of the representation comprise all or part of the following list:
• a three-dimensional position of the representation of the face;
• a three-dimensional orientation of the representation of the face;
• a size of the model of the pair of spectacles;
• a size of the model of the face;
• a relative three-dimensional position between the model of the pair of spectacles and the model of the face;
• a relative three-dimensional orientation between the model of the pair of spectacles and the model of the face;
• one or more parameter(s) of the configuration of the model of the pair of spectacles;
• one or more parameter(s) of the configuration of the model of the face;
• one or more parameter(s) of the camera.

12. Tracking method according to the preceding claim, comprising steps of:
• detection of a plurality of points of the face in a first image of the video stream;
• initialization of the set of parameters of the model of the face with respect to the image of the face in said first initial image;
• detection of a plurality of points of a pair of spectacles worn by the face of the individual in a second image of the video stream, referred to as second initial image, the second initial image being either subsequent to or prior to the first initial image in the video stream, or identical to the first image in the video stream;
• initialization of the set of parameters of the model of the pair of spectacles with respect to the image of the pair of spectacles in said second initial image.

13. Tracking method according to the preceding claim, wherein the initialization of the parameters of the model of the face is implemented by means of a deep learning method of analyzing all or some of the detected points of the face.

14. Tracking method according to the preceding claim, wherein the deep learning method also determines an initial position of the model of the face in the three-dimensional reference frame.

15. Tracking method according to any one of the preceding claims, also comprising a step of determining a scale of the image of the pair of spectacles worn by the face of the individual by means of a dimension in the image of an element of known size of the pair of spectacles.

16. Tracking method according to the preceding claim, wherein the determination of the scale is implemented by means of a prior recognition of the pair of spectacles worn by the face of the individual.

17. Tracking method according to any one of the preceding claims, wherein images acquired by a second image-acquisition device are used to evaluate the parameters of the representation.

18. Tracking method according to any one of the preceding claims, wherein the model of the pair of spectacles of the representation corresponds to a prior modelling of said pair of spectacles, and varies solely in deformation.

19. Augmented reality method comprising steps of:
• acquiring at least one stream of images of an individual wearing a pair of spectacles on their face by means of at least one image-acquisition device;
• tracking of the face of the individual by a tracking method according to any one of claims 1 to 18, a position and an orientation of a representation of the face;
• modifying of all or some of the images of said or of one of said image streams, referred to as main video stream, acquired by the image-acquisition device or by one of the image-acquisition devices, referred to as main image-acquisition device, by means of the representation of the face superimposed in real time on the face of the individual on the main video stream;
• displaying on a screen said previously modified main video stream.

20. Electronic device including a computer memory storing instructions of a method according to any one of the preceding claims.
